# EUROPEAN PATENT SPECIFICATION

(11) **EP 3 909 634 B1**
(45) Date of publication and mention of the grant of the patent: **19.07.2023**
(21) Application number: 21171510.7
(22) Date of filing: 30.04.2021
(51) Int. Cl.: A61M 25/01

(54) **DEFLECTABLE CATHETER SYSTEMS**
SYSTEME MIT ABLENKBAREM KATHETER
SYSTÈMES DE CATHÉTER ORIENTABLE

(30) Priority: 01.05.2020 US 202063018721 P; 06.04.2021 US 202163171263 P
(43) Date of publication of application: 17.11.2021
(73) Proprietor: Cook Medical Technologies LLC, Bloomington, IN 47404 (US)
(72) Inventor: BOOKER, Robert, Vandergrift, PA 15690 (US); HARRIS, William, Cowlinge, Suffolk CB8 9QG (GB); NORRIS, Deborah, Ely CB7 4TN (GB); BARTON, Rupert, Cheltenham GL50 3ED (GB); PENROSE, Frances, Ely CB7 4HJ (GB); HILES, John, Great Sutton CH66 4UT (GB)
(74) Representative: Warren, Caroline Elisabeth

(56) References cited:
- WO-A1-2019/005428
- WO-A2-2013/069019
- US-A- 5 254 088

## Description

### Field

The disclosure relates generally to the field of medical devices and methods of using a medical device. More particularly, the disclosure relates to deflectable catheter systems and methods of using a deflectable catheter system.

### Background

Medical devices such as implanted cardiac pacing systems, such as pacemakers and defibrillators, generally employ an implanted power source (e.g., pulse generator) and one more leads. The leads are attached to the pulse generator and the heart and include electrodes in direct contact with heart tissue to convey electrical stimulation of the heart muscle. Proper placement of the leads in the body is necessary to assure delivery of the electrical stimuli and accomplish electrical stimulation of the heart muscle. As a result, the leads are disposed within various portions of the body. For example, a lead can be positioned within an artery, a vein, or a chamber of the heart.

Subsequent to implantation, the body may react to the implanted cardiac pacing system by forming scar tissue along a lead and its associated electrode. While leads are generally designed to be implanted permanently, there are instances in which it may be necessary to remove and/or replace a lead, such as when the patient develops an infection or the pacemaker or cardiac defibrillator has malfunctioned. The presence of any scar tissue around the lead and its associated electrode increases the difficulty associated with removing the lead.

Various lead removal devices have been developed to increase the efficiency of extracting an implanted lead. For example, some lead removal devices include a sheath and a dissecting blade disposed on the distal end of the sheath. The sheath is advanced over the lead and the dissecting blade assists with separating any scar tissue from the lead such that it can be removed. However, these devices have drawbacks. For example, these devices do not include a mechanism for navigating the sheath through the tortuous anatomy of the body and to the vessel within which the lead is positioned, which increases the time required to complete the lead removal procedure and the risks associated with the procedure.

A need exists, therefore, for new and useful deflectable catheter systems and methods of using a deflectable catheter system.

WO 2013/069019 describes an apparatus for percutaneous access to a patient's body comprising a first steerable tube (12), shaped to define a first lumen, and a first coupling (152) at a longitudinal site of the first tube; and a second steerable tube (14), shaped to define a second lumen and a second coupling (154), the second coupling being intracorporeally couplable to the first coupling, the apparatus having (A) an unlocked state in which the second tube is rotatable within the first lumen, and (B) a locked state in which the second coupling is coupled to the first coupling, and rotation of the second tube is inhibited.

### Summary of Selected Example Embodiments

The invention is set out in the appended claims. Any methods disclosed do not form part of the scope of invention except where the method is not a method of treatment of the human or animal body by surgery or therapy.

Various example deflectable catheter systems and methods of using a deflectable catheter system are described herein. Aspects of the invention are set out in the dependent claims and preferred features are set out in the dependent claims. Preferred features of one aspect may be applied to other aspects.

An example deflectable catheter system includes a housing, an orienting member, an actuator, a first elongate member, a second elongate member, and a wire member. The housing defines a passageway. The orienting member is attached to the housing and is moveable relative to the housing between an orienting member first position and an orienting second position. The actuator is attached to the orienting member and is moveable between an actuator first position and an actuator second position. The first elongate member is attached to the orienting member and defines a lumen. The first elongate member is moveable between a first, substantially straight configuration when the actuator is in the actuator first position and a second, curved configuration when the actuator is in the actuator second position. The second elongate member is disposed within the passageway defined by the housing and extends through the lumen defined by the first elongate member. The second elongate member is rotatable relative to the first elongate member. The wire member has a first end attached to the actuator and a second end attached to the first elongate member.

Another example deflectable catheter system includes a housing, an orienting member, an actuator, a first elongate member, a second elongate member, and a wire member. The housing defines a passageway. The orienting member is attached to the housing and is moveable relative to the housing between an orienting member first position and an orienting member second position. The actuator is attached to the orienting member and is moveable between an actuator first position and an actuator second position. The actuator has a main body and a hub disposed within the main body. The hub mates with the main body of the actuator such that movement of the actuator between the actuator first position and the actuator second position results in movement of the hub between a first position and a second position. The first elongate member is attached to the orienting member and defines a lumen. The first elongate member is moveable between a first, substantially straight configuration when the actuator is in the actuator first position and a second, curved configuration when the actuator is in the actuator second position. The second elongate member is disposed through the passageway defined by the housing and extends through the lumen defined by the first elongate member. The second elongate member is rotatable relative to the housing and the first elongate member. The second elongate member is moveable between a first, substantially straight configuration when the actuator is in the actuator first position and a second, curved configuration when the actuator is in the actuator second position. The wire member is disposed between the first elongate member and the second elongate member. The wire member has a first end attached to the hub of the actuator and a second end attached to the first elongate member. Movement of the orienting member between the orienting member first position and the orienting second position results in rotation of the first elongate member and wire member relative to the second elongate member.

An example method of using a deflectable catheter system to remove a medical device attached to a portion of a body of an animal where the medical device is disposed within a bodily passage of the body includes: obtaining a deflectable catheter system; introducing a portion of the medical device into a lumen defined by a second elongate member of the deflectable catheter system; applying a proximally-directed force on the medical device while maintaining the position of the deflectable catheter system until the medical device is disposed proximal to a proximal end of the second elongate member; applying a distally-directed force on the deflectable catheter system while applying proximally-directed force on the medical device such that the second elongate member is introduced into the bodily passage; continuing the application of a distally-directed force on the deflectable catheter system while applying proximally-directed force on the medical device such that the second elongate member is advanced into the bodily passage; applying a proximally-directed force on a trigger of the deflectable catheter system while maintaining the position of a handle of the deflectable catheter system to rotate the second elongate member relative to a first elongate member of the deflectable catheter system; stopping the application of a proximally-directed force on the trigger; applying a distally-directed force on the deflectable catheter system while applying a proximally-directed force on the medical device such that the second elongate member is advanced into the bodily passage; applying torque to an orienting member of the deflectable catheter system while maintaining the position of the housing to move the orienting member, the first elongate member, and a wire member of the deflectable catheter system relative to the second elongate member; moving the actuator from the actuator first position to the actuator second position such that the first and second elongate members move from the first substantially, straight configuration to the second curved configuration; applying a distally-directed force on the deflectable catheter system while applying proximally-directed force on the medical device such that the second elongate member is advanced into the bodily passage; moving the actuator from the actuator second position to the actuator first position such that the first and second elongate members move from the second, curved configuration to the first substantially, straight configuration; applying a distally-directed force on the deflectable catheter system while applying proximally-directed force on the medical device such that the second elongate member is advanced to the distal end of the medical device; applying a proximally-directed force on the medical device while maintaining the position of the deflectable catheter system until the medical device is withdrawn from said bodily passage; applying a proximally-directed force on the deflectable catheter system until the second elongate member is withdrawn from the bodily passage.

Another example method of using a deflectable catheter system to retrieve a medical device includes: obtaining a deflectable catheter system comprising: a housing having a trigger, a handle, and defining a passageway; an orienting member attached to the housing and moveable relative to the housing between an orienting member first position and an orienting member second position; an actuator attached to the orienting member and moveable between an actuator first position and an actuator second position; a first elongate member attached to the orienting member and defining a lumen, the first elongate member moveable between a first, substantially straight configuration when the actuator is in the actuator first position and a second, curved configuration when the actuator is in the actuator second position; a second elongate member disposed through the passageway defined by the housing and extending through the lumen defined by the first elongate member, the second elongate member rotatable relative to the housing and the first elongate member, the second elongate member moveable between a first, substantially straight configuration when the actuator is in the actuator first position and a second, curved configuration when the actuator is in the actuator second position, the second elongate member having a proximal end, a distal end having a plurality of teeth, and defining a lumen; and a wire member having a first end attached to the actuator and a second end attached to the first elongate member. Movement of the orienting member between the orienting member first position and the orienting second position results in rotation of the first elongate member and wire member relative to the second elongate member. The method includes introducing a portion of said medical device into the lumen defined by the second elongate member; applying a proximally-directed force on said medical device while maintaining the position of the deflectable catheter system until said medical device is disposed proximal to the proximal end of the second elongate member; applying a distally-directed force on the deflectable catheter system while applying proximally-directed force on said medical device; continuing the application of a distally-directed force on the deflectable catheter system while applying proximally-directed force on said medical device; applying a proximally-directed force on the trigger while maintaining the position of the handle to rotate the second elongate member relative to the first elongate member; stopping the application of a proximally-directed force on the trigger; applying a distally-directed force on the deflectable catheter system while applying a proximally-directed force on said medical device; applying torque to the orienting member while maintaining the position of the housing to move the orienting member, the first elongate member, and the wire member relative to the second elongate member; moving the actuator from the actuator first position to the actuator second position such that each of the first and second elongate members move from the first substantially, straight configuration to the second curved configuration; applying a distally-directed force on the deflectable catheter system while applying proximally-directed force on said medical device; moving the actuator from the actuator second position to the actuator first position such that each of the first and second elongate members move from the second, curved configuration to the first substantially, straight configuration; applying a distally-directed force on the deflectable catheter system while applying proximally-directed force on said medical device such that the second elongate member is advanced to the distal end of said medical device; applying a proximally-directed force on said medical device while maintaining the position of the deflectable catheter system; and applying a proximally-directed force on the deflectable catheter system.

The methods described herein may be used in a training scenario, for example outside the body or using a cadaver or simulated bodily passages used to learn the methods described.

Additional understanding of these example deflectable catheter systems and methods of using a deflectable catheter system can be obtained by review of the detailed description, below, and the appended drawings.

### Brief Description of the Drawings

FIG. 1 is a partial side view of an example deflectable catheter system. The first elongate member is in the first, substantially straight configuration.
FIG. 1A is a partial side view of the distal end of the deflectable catheter system illustrated in FIG. 1. The first elongate member is in the second, curved configuration.
FIG. 2 is a partial cross-sectional view of the deflectable catheter system illustrated in FIG. 1 taken along the lengthwise axis of the housing.
FIG. 3 is a perspective view of the main body of the actuator of the deflectable catheter system illustrated in FIG. 1.
FIG. 4 is a partially broken away partial perspective view of the housing of the deflectable catheter system illustrated in FIG. 1.
FIG. 5 is another partially broken away partial perspective view of the distal end of the housing of the deflectable catheter system illustrated in FIG. 1.
FIG. 6 is a partial perspective view of a portion of the first elongate member and wire housing of the deflectable catheter system illustrated in FIG. 1.
FIG. 7 is another partial perspective view of a portion of the first elongate member and wire housing of the deflectable catheter system illustrated in FIG. 1.
FIG. 8 is a partial side view of a portion of the first elongate member of the deflectable catheter system illustrated in FIG. 1.
FIG. 9 is a sectional view of the first elongate member of the deflectable catheter system illustrated in FIG. 8 taken along line 9-9 shown in FIG. 8.
FIG. 10 is a partial perspective view of the distal end of the deflectable catheter system illustrated in FIG. 1.
FIG. 11 is a partial perspective view of a medical device adapted to receive a deflectable catheter system.
FIG. 12 is a partial side view of the medical device illustrated in FIG. 11.
FIG. 13 is a perspective view of another example deflectable catheter system.
FIG. 14 is a partially broken away partial perspective view of a portion of the deflectable catheter system illustrated in FIG. 13.
FIG. 15 is a cross-sectional view of a portion of the deflectable catheter system illustrated in FIG. 13 taken along the lengthwise axis of the housing.
FIG. 16 is a partial side view of a portion of the deflectable catheter system illustrated in FIG. 13. The locking mechanism is in the first position and the first portion of the housing is in the housing first portion.
FIG. 17 is a partial perspective view of the distal end of the deflectable catheter system illustrated in FIG. 13. The distal end of the second elongate member is disposed outside of the lumen of the first elongate member.
FIG. 18 is a partial side view of a portion of the deflectable catheter system illustrated in FIG. 13. The locking mechanism is in the second position and the first portion of the housing is in the housing second position.
FIG. 19 is a partial perspective view of the distal end of the deflectable catheter system illustrated in FIG. 13. The distal end of the second elongate member is disposed within the lumen of the first elongate member.
FIG. 20 is a schematic illustration of an example method of using a deflectable catheter system.
FIG. 21 is a perspective view of another example deflectable catheter system. Each of the first elongate member and the second elongate member is in the first, substantially straight configuration.
FIG. 22 is a side view of the deflectable catheter system illustrated in FIG. 21. Each of the first elongate member and the second elongate member is illustrated in the first, substantially straight configuration, a configuration between the first, substantially straight configuration and the second, curved configuration, and in the second, curved configuration. The third elongate member is omitted for clarity.
FIG. 23 is a magnified view of area A-A shown in FIG. 22.
FIG. 24 is a partial side view of the deflectable catheter system illustrated in FIG. 21.
FIG. 25 is a partial top view of the deflectable catheter system illustrated in FIG. 21.
FIG. 26 is a partial bottom view of the deflectable catheter system illustrated in FIG. 21.
FIG. 27 is a distal end view of the deflectable catheter system illustrated in FIG. 21.
FIG. 28 is a proximal end view of the deflectable catheter system illustrated in FIG. 21.
FIG. 29 is a partially broken away partial perspective view of the deflectable catheter system illustrated in FIG. 21.
FIG. 30 is a magnified view of area B-B illustrated in FIG. 29.
FIG. 31 is a partial perspective view of the second elongate member, the orienting member, the actuator, and the wire member of the deflectable catheter system illustrated in FIG. 21.
FIG. 32 is a partial cross-sectional view of the deflectable catheter system illustrated in FIG. 21 taken along the lengthwise axis of the housing.
FIG. 33 is a perspective view of the retaining member of the deflectable catheter system illustrated in FIG. 21.
FIG. 34 is a perspective view of the first portion of the orienting member of the deflectable catheter system illustrated in FIG. 21.
FIG. 35 is a perspective view of the second portion of the orienting member of the deflectable catheter system illustrated in FIG. 21.
FIG. 36 is an end view of the second portion of the orienting member illustrated in FIG. 35.
FIG. 37 is a perspective view of the hub the actuator of the deflectable catheter system illustrated in FIG. 21.
FIG. 38 is an end view of the hub illustrated in FIG. 37.
FIG. 39 is a perspective view of the first portion of the actuator main body of the deflectable catheter system illustrated in FIG. 21.
FIG. 40 is a perspective view of the second portion of the actuator main body of the deflectable catheter system illustrated in FIG. 21.
FIG. 41 is a partial side view of the distal end of the deflectable catheter system illustrated in FIG. 21.
FIG. 42 is a partial cross-sectional view of the distal end of the deflectable catheter system illustrated in FIG. 21 taken along the lengthwise axis of the first elongate member.
FIG. 43 is a partial perspective view of the distal end of the deflectable catheter system illustrated in FIG. 21. The first elongate member has been removed for clarity.
FIG. 44 is an exploded view of a portion of the second elongate member, the ring member, the retaining member, and the distal tip.
FIG. 45 is a partially broken away partial perspective view of a distal end of another example deflectable catheter system.
FIG. 46 is a partial perspective view of a distal end of another example deflectable catheter system.
FIG. 47 is a partial side view of the deflectable catheter system illustrated in FIG. 46.
FIG. 48 is a partial cross-sectional view of the deflectable catheter system illustrated in FIG. 46 taken along the lengthwise axis of the first elongate member.
FIG. 49 is a partial perspective view of another example distal tip that can be included in a deflectable catheter system.
FIG. 50 is a partial perspective view of another example distal tip that can be included in a deflectable catheter system.
FIG. 51 is a partial perspective view of another example distal tip that can be included in a deflectable catheter system.
FIG. 52 is a partial perspective view of another example distal tip that can be included in a deflectable catheter system.
FIG. 53 is a cross-sectional view of the distal tip illustrated in FIG. 52 taken along the lengthwise axis of the distal tip.
FIG. 54 is a partial perspective view of another example distal tip that can be included in a deflectable catheter system.
FIG. 55 is a cross-sectional view of the distal tip illustrated in FIG. 54 taken along the lengthwise axis of the distal tip.
FIG. 56 is a partial perspective view of another example distal tip that can be included in a deflectable catheter system.
FIG. 57 is a magnified view of area C-C illustrated in FIG. 56.
FIG. 58 is a partial perspective view of a distal end of another example deflectable catheter system.
FIG. 59 is a partial cross-sectional view of the deflectable catheter system illustrated in FIG. 58 taken along the lengthwise axis of the first elongate member.
FIG. 60 is a partial perspective view of another example handle that can be included in a deflectable catheter system.
FIG. 61 is a partial perspective view of another example handle that can be included in a deflectable catheter system.
FIG. 62 is a partial perspective view of another example handle that can be included in a deflectable catheter system.
FIG. 63 is a partially broken away partial perspective view of a distal end of another example deflectable catheter system.
FIG. 64 is a partially exploded partial perspective view of the deflectable catheter system illustrated in FIG. 63.
FIG. 65 is an exploded cross-sectional view of the distal tip and ring member of the deflectable catheter system illustrated in FIG. 63 taken along the lengthwise axis of the distal tip.
FIG. 66 is a cross-sectional view of the distal tip and ring member of the deflectable catheter system illustrated in FIG. 63 taken along the lengthwise axis of the distal tip.
FIG. 67 is a partial perspective view of another example deflectable catheter system.
FIG. 68 is a partial cross-sectional view of the deflectable catheter system illustrated in FIG. 67 taken along the lengthwise axis of the first elongate member.
FIG. 69 is a magnified view of area D-D illustrated in FIG. 68.
FIG. 70 is a partial perspective view of a portion of the deflectable catheter system illustrated in FIG. 67.
FIG. 71 is a schematic illustration of another example method of using a deflectable catheter system.

### Detailed Description of Selected Examples

The following detailed description and the appended drawings describe and illustrate various example embodiments of deflectable catheter systems and methods of using a deflectable catheter system. The description and illustration of these examples are provided to enable one skilled in the art to make and use a deflectable catheter system and practice a method of using a deflectable catheter system. They are not intended to limit the scope of the claims in any manner. The invention is capable of being practiced or carried out in various ways and the examples described and illustrated herein are merely selected examples of the various ways of practicing or carrying out the invention and are not considered exhaustive.

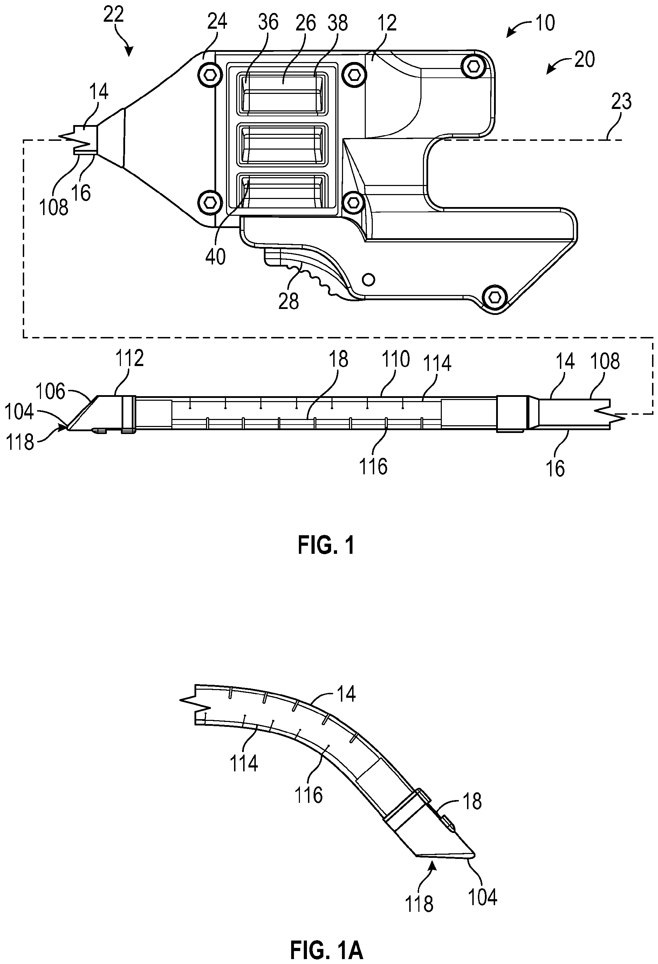

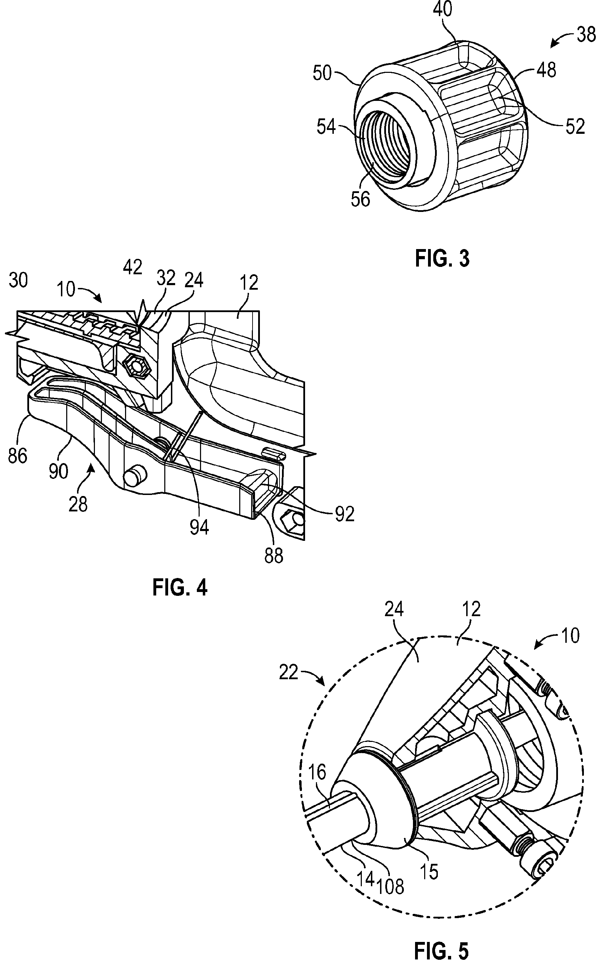

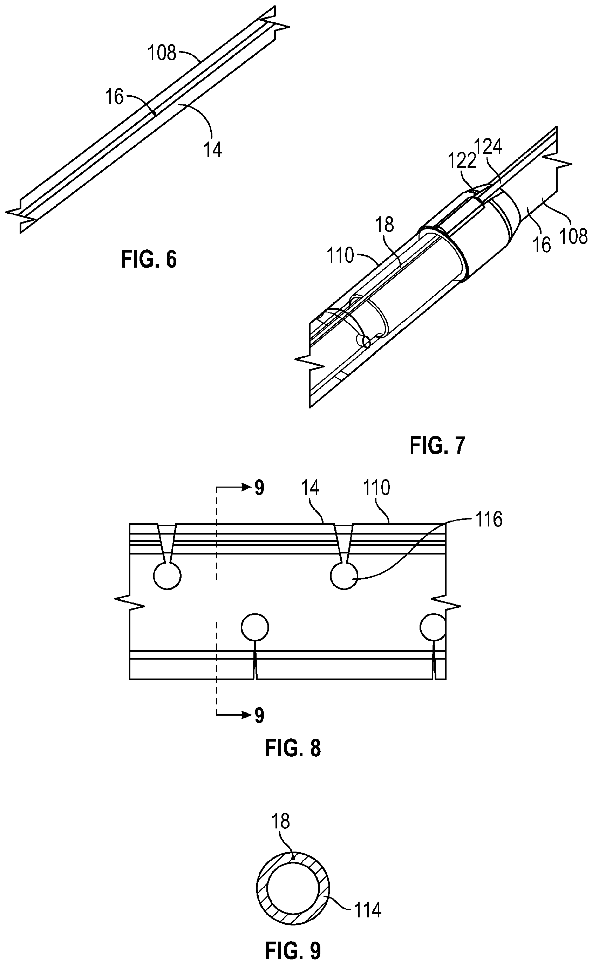

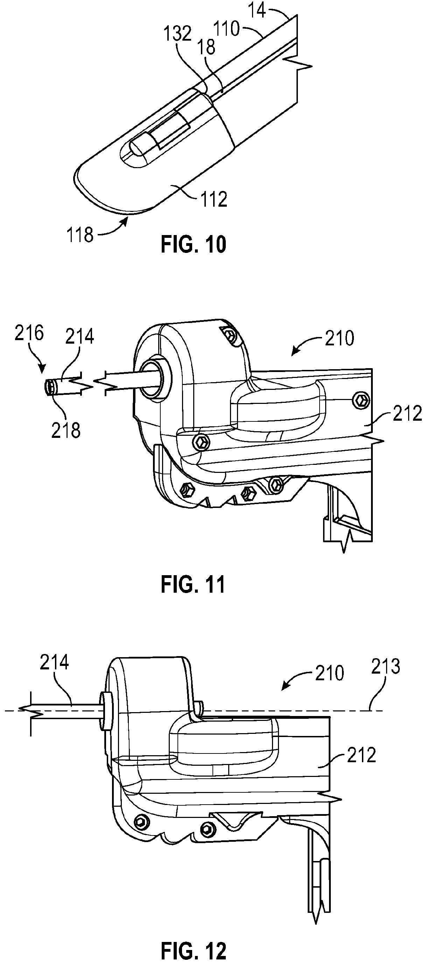

FIGS. 1, 1A, 2, 3, 4, 5, 6, 7, 8, 9, and 10 illustrate an example deflectable catheter system 10. The deflectable catheter system 10 is adapted to be releasably attached to a medical device, such as the medical device 210 illustrated in FIGS. 11 and 12. The deflectable catheter system 10 has a housing 12, a first elongate member 14, an orienting member 15, a wire housing 16, a wire member 18, an actuator 26, and a locking mechanism 28.

The housing 12 has a proximal end 20, a distal end 22, a lengthwise axis 23, and a main body 24. The main body 24 of the housing 12 defines a chamber 30, a passageway 32 that extends through the chamber 30, a recess 33, and first and second notches 34. The lengthwise axis 23 of the housing 12 extends through the passageway 32 defined by the main body 24.

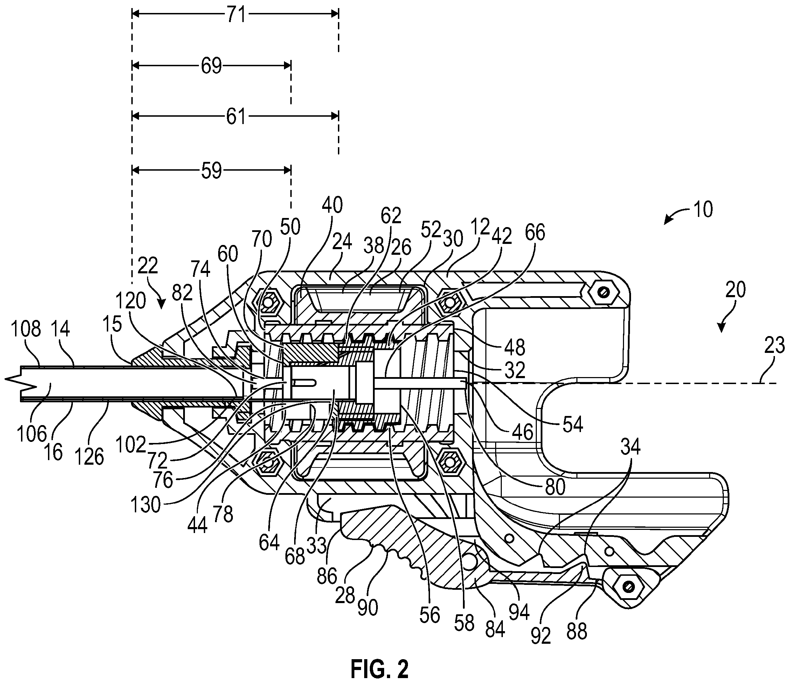

The actuator 26 is moveably disposed within the chamber 30 defined by the main body 24 of the housing 12. The actuator 26 is moveable between an actuator first position, as shown in FIG. 2, and an actuator second position. Any suitable actuator can be included in a deflectable catheter system and selection of a suitable actuator can be based on various considerations, such as the structural arrangement of a housing a deflectable catheter system. Examples of actuators considered suitable to include in a deflectable catheter system include rotatable actuators, linear actuators, and any other actuator considered suitable for a particular embodiment. In the illustrated embodiment, the actuator 26 is a rotatable actuator 36. Specifically, in the illustrated embodiment, the actuator 26 is an index wheel 38.

In the illustrated embodiment, the actuator 26 has a main body 40, a first hub 42, a second hub 44, and first and second rails 46. The main body 40 of the actuator 26 is moveable between the actuator first position and the actuator second position. The main body 40 of the actuator 26 has a first end 48, a second end 50, and defines a plurality of recesses 52, a passageway 54, and a first thread 56. The plurality of recesses 52 extend into the main body 40 and provide a mechanism for a user to move the actuator between its first and second positions and provide tactile feedback regarding the position of the main body 40. The passageway 54 extends from the first end 48 to the second end 50 and, in the illustrated embodiment, is coaxial with the passageway 32 defined by the main body 24 of the housing 12. The first thread 56 extends into the main body 40 of the actuator 26 within the passageway 54 and mates with the second thread 62 defined by the first hub 42. While FIG. 2 illustrates a cross-sectional view of the deflectable catheter system 10, it is to be understood that the portion of the catheter system not illustrated mirrors that of the portion illustrated, unless otherwise described.

Each of the first hub 42, the second hub 44, and the first and second rails 46 is disposed within the passageway 54 defined by the main body 40 of the actuator 26. The first hub 42 has a first end 58, a second end 60, and defines a second thread 62, a passageway 64, and first and second recesses 66. The second thread 62 extends from an exterior surface of the first hub 42 and mates with the first thread 56 defined by the main body 40 of the actuator 26. The passageway 64 extends from the first end 58 to the second end 60 of the first hub 42. Each of the first and second recesses 66 extends from the first end 58 to the second end 60 of the first hub 42 and is sized to receive a rail of the first and second rails 46, as described herein, to allow the first hub 42 to travel between first and second positions within the main body 40 of the actuator 26 and to prevent rotation of the first hub 42 about the lengthwise axis 23 during use. As the main body 40 of the actuator 26 is moved between the actuator first position and the actuator second position, the threads 56, 62 interact with one another to achieve movement of the first hub 42 within the passageway 54 defined by the actuator 26. The first hub 42 is moveable along the first and second rails 46 between a first position and a second position. The first hub 42 is disposed in the first position, as shown in FIG. 2, when the actuator 26 is in the actuator first position and the first hub 42 is disposed in the second position when the actuator 26 is in the second position. In the first position, the first hub 42 is disposed a first distance 59 from the distal end 22 of the housing 12. In the second position, the first hub 42 is disposed a second distance 61 from the distal end 22 of the housing 12, which is different than the first distance 59. In the illustrated embodiment, the second distance 61 is greater than the first distance 59.

The second hub 44 is disposed adjacent to, is attached to, and directly contacts the first hub 42 and is disposed between the first hub 42 and the housing 12. In alternative embodiments, a first hub and a second hub can comprise a single, unitary component or a second hub can be separate from and not directly contact a first hub. The second hub 44 has a first end 68, a second end 70, and defines a passageway 72, first and second recesses 74, a third recess 76, and a fourth recess 78. The passageway 72 extends from the first end 68 to the second end 70 of the second hub 44. Each of the first and second recesses 74 extends from the first end 68 to the second end 70 of the second hub 44 and is sized to receive a rail of the first and second rails 46, as described herein, to allow the second hub 44 to travel between its first and second positions within the main body 40 of the actuator 26 and to prevent rotation of the second hub 44 about the lengthwise axis 23 during use, as described herein. The third recess 76 extends from the second end 70 toward the first end 68 of the second hub 44, from an exterior surface of the second hub 44 toward the passageway 72 defined by the second hub 44, and is sized to receive a portion of the wire member 18, as described herein, to accomplished movement of the first elongate member 14. The fourth recess 78 is in communication with the third recess 76, extends from an exterior surface of the second hub 44 toward the passageway 72 defined by the hub 44, and is sized to receive a portion of the wire member 18, as described herein. The fourth recess 78 is sized to receive a portion of an attachment member (e.g., anchor, set screw) to accomplished attachment of the wire member 18 to the second hub 44. As the main body 40 of the actuator 26 is moved between the actuator first position and the actuator second position, the threads 56, 62 interact with one another to achieve movement of the first hub 42 and the second hub 44 within the passageway 54 defined by the actuator 26. The second hub 44 is moveable along the first and second rails 46 between a first position and a second position. The second hub 44 is disposed at a first location, as shown in FIG. 2, when the actuator 26 is in the actuator first position and at a second location when the actuator 26 is in the actuator second position. In the first position, the second hub 44 is disposed a first distance 69 from the distal end 22 of the housing 12. In the second position, the second hub 44 is disposed a second distance 71 from the distal end 22 of the housing 12, which is different than the first distance 69. In the illustrated embodiment, the second distance 71 is greater than the first distance 69.

One of the first and second rails 46 is disposed within a recess 66 defined by the first hub 42 and a recess 74 defined by the second hub 44. Each of the first and second rails 46 has a first end 80 and a second end 82. Each of the first and second rails 46 provides a track upon which the first and second hubs 42, 44 can move relative to the housing 12 as the actuator 26 is moved between the actuator first position and the actuator second position.

The locking mechanism 28 is adapted to releasably lock the housing 12 in a housing first position and a housing second position when the deflectable catheter system 10 is releasably attached to a medical device. Any suitable locking mechanism can be included in a deflectable catheter system and selection of a suitable locking mechanism can be based on various considerations, such as the structural arrangement of a housing a deflectable catheter system. Examples of locking mechanisms considered suitable to include in a deflectable catheter system include spring activated locking mechanisms, depressible buttons, and any other locking mechanism considered suitable for a particular embodiment. In the illustrated embodiment, the locking mechanism 28 comprises a spring activated main body 84 that has a first end 86, a second end 88, a depressible portion 90, a projection 92, and a spring 94. When a force is applied to the depressible portion 90 and directed toward the housing 12, a portion of the main body 84 is moved within the recess 33 defined by the main body 24 of the housing 12. The main body 84 is moveable between first and second positions. The spring 94 biases the main body 84 to the first position, as shown in FIG. 2. The projection 92 extends from the main body 84 and is sized such that it can be disposed within one of the first and second notches 34 defined by the main body 24 of the housing 12 and such that the housing 12 can be moved between first and second positions when attached to a medical device.

The first elongate member 14 is attached to the housing 12 using the orienting member 15 which is rotatably attached to the housing 12 and moveable relative to the housing between first and second positions, as described herein. In alternative embodiments, however, a first elongate member can be directly attached to a housing. The first elongate member 14 has a proximal end 102, a distal end 104, and a lumen 106. The first elongate member 14 is moveable between a first, substantially straight configuration when the actuator 26 is in the actuator first position, as shown in FIG. 1, and a second, curved configuration when the actuator 26 is in the actuator second position, as shown in FIG. 1A (e.g., such that the first elongate member can pass from the innominate vein down to the superior vena cava and eventually the heart during use). The first elongate member 14 has a first portion 108, a second portion 110, and a cap 112. The first portion 108 extends from the proximal end 102 toward the distal end 104 and is formed of a first material that has a first flexibility. The second portion 110 extends from the first portion 108 toward the distal end 104 and is formed of a second material that has a second flexibility. The second portion 110 has a wall 114 that has a thickness. The second material that forms the second portion 110 is relatively more flexible than the first material that forms the first portion 108. In the illustrated embodiment, the second portion 110 includes a plurality of notches 116 to assist with deflection of the first elongate member 14. The cap 112 extends from the second portion 110 to the distal end 104 of the first elongate member 14 and has a tapered distal end 118. In the illustrated embodiment, the wire member 18 has a second end 132 attached to the first elongate member 14. In the illustrated embodiment, the second end 132 of the wire member 18 is attached to the cap 112.

The wire housing 16 is attached to the housing 12 and has a proximal end 120, a distal end 122, and a main body 124 that defines a lumen 126 sized to receive a portion of the wire member 18. The wire housing 16 extends from the housing 12 to the second portion 112 of the first elongate member 14. In alternative embodiments, a wire housing can be omitted from a deflectable catheter system. A wire housing included in a deflectable catheter system can have any suitable structural arrangement, such as a closed coil spring, a Bowden cable, and any other structural arrangement considered suitable for a particular embodiment.

The wire member 18 has a first end 130 and a second end 132. The first end 130 of the wire member 18 is attached to the second hub 44 and is partially disposed within the third recess 76 and the fourth recess 78. The second end 132 is attached to the cap 112. The wire member 18 extends from the first end 130, through a portion of the actuator 26, through a portion of the housing 12, through the lumen 126 of the wire housing 16, through the second portion 110 of the first elongate member 14, to the cap 112. The wire member 18 is disposed within the wall 114 of the second portion 110 of the first elongate member 14.

FIGS. 11 and 12 illustrate an example medical device 210 on which a deflectable catheter system, such as those described herein, can be attached. The medical device 210 has a medical device housing 212 and a second elongate member 214 disposed through the medical device housing 212 and is rotatably attached to the medical device housing 212 using bearings, as described in more detail herein. The medical device housing 212 has a medical device lengthwise axis 213. The medical device elongate member 214 has a distal end 216 that has a plurality of teeth 218. When attached to the medical device 210, the housing 12 of the deflectable catheter system 10 is moveable relative to the medical device housing 212 axially along the medical device lengthwise axis 213 between a housing first position and a housing second position. In the housing first position, the distal end 216 of the second elongate member 214 is disposed outside of the lumen 106 of the first elongate member 14. In the housing second position, the distal end 218 of the second elongate member 214 is entirely disposed within the lumen 106 of the first elongate member 14.

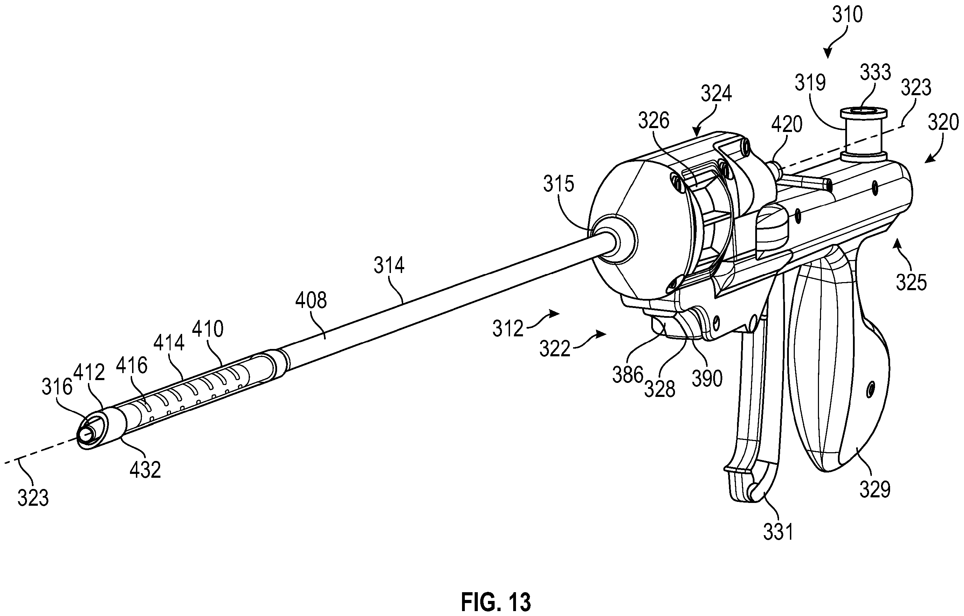

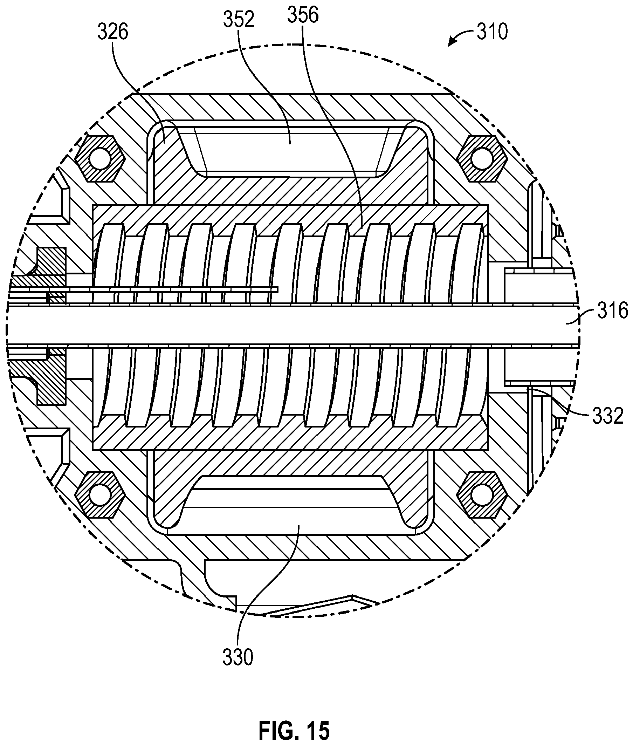

FIGS. 13, 14, 15, 16, 17, 18, and 19 illustrate another example deflectable catheter system 310. The deflectable catheter system 310 is similar to the deflectable catheter system 10 illustrated in FIGS. 1, 2, 3, 4, 5, 6, 7, 8, 9, and 10 and described above, except as detailed below. In the illustrated embodiment, the deflectable catheter system 310 has a housing 312, a first elongate member 314, an orienting member 315, a second elongate member 316, a wire member 318, and a capstan 319.

The housing 312 has a proximal end 320, a distal end 322, a lengthwise axis 323, a first portion 324, a second portion 325, an actuator 326, and a locking mechanism 328. The first portion 324 of the housing 312 defines a chamber 330, a passageway 332 that extends through the chamber 330, a recess 333, and first and second notches 334. The lengthwise axis 323 of the housing 312 extends through the passageway 332 defined by the first portion 324. The second portion 325 has a handle 329, a trigger 331, and a post 333. The trigger 331 is moveable between a first position and a second position. A drive mechanism 341 housed within the second portion 325 is in an inactive state when the trigger 331 is in the first position and is in an active state when the trigger 331 is moved between the first position and the second position. The second elongate member 316 rotates about the lengthwise axis 323 of the housing 312 when the drive mechanism 341 is in the active state.

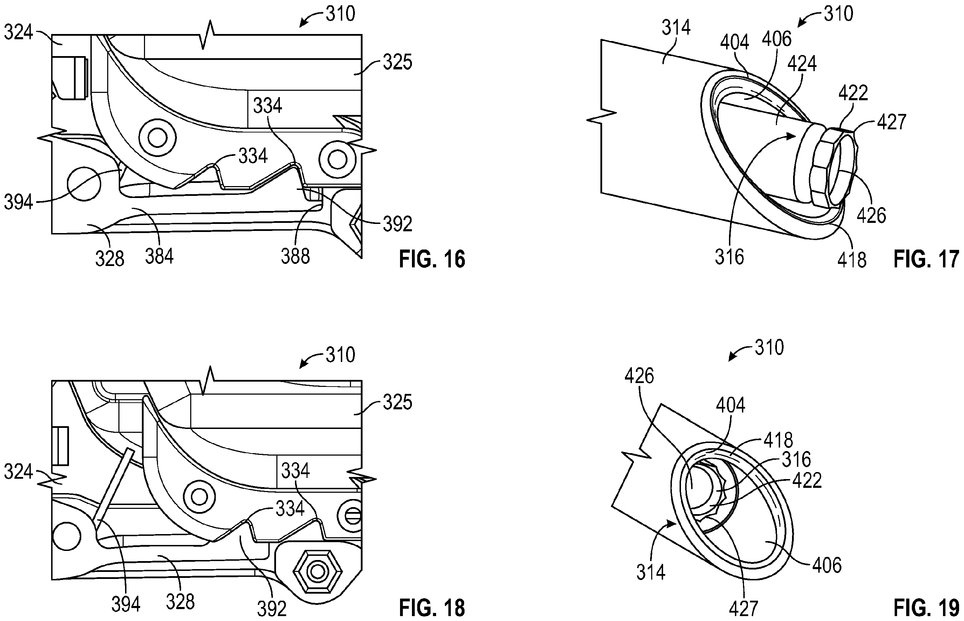

The first portion 324 is moveable relative to the second portion 325 axially along the housing lengthwise axis 323 between a housing first position and a housing second position. In the housing first position, as shown in FIG. 16, the distal end 422 of the second elongate member 316 is disposed outside of the lumen 406 of the first elongate member 314, as shown in FIG. 17. In the housing second position, as shown in FIG. 18, the distal end 422 of the second elongate member 316 is entirely disposed with the lumen 406 of the first elongate member 314, as shown in FIG. 19. While illustrated as fixedly attached to one another, a first portion of a housing can be releasably attached to a second portion of a housing.

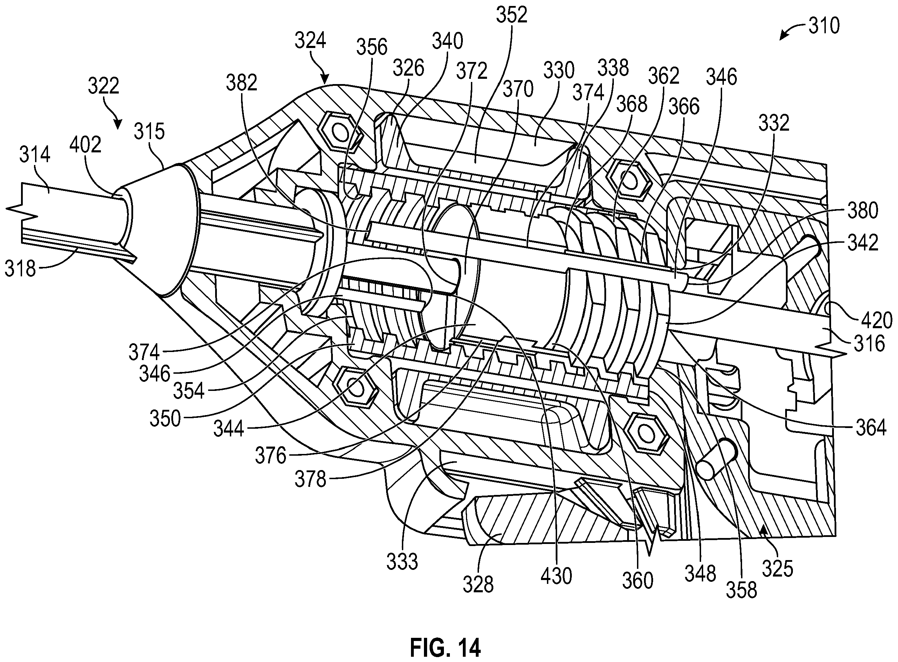

The actuator 326 is moveably disposed within the chamber 330 defined by the first portion 324 of the housing 312. The actuator 326 is moveable between an actuator first position, as shown in FIG. 13, and an actuator second position, as shown in FIG. 14. In the illustrated embodiment, the actuator 326 is an index wheel 338. The actuator 326 is disposed on the first portion 324 of the housing 312 and has a main body 340, a first hub 342, a second hub 344, and first and second rails 346. The main body 340 of the actuator 326 has a first end 348, a second end 350, and defines a plurality of recesses 352, a passageway 354, and a first thread 356. The plurality of recesses 352 extend into the main body 340 and provide a mechanism for a user to move the actuator between its first and second positions and provide tactile feedback regarding the position of the main body 340. The passageway 354 extends from the first end 348 to the second end 350 and, in the illustrated embodiment, is coaxial with the passageway 332 defined by the first portion 324 of the housing 312. The first thread 356 extends into the passageway 354 and mates with the second thread 362 defined by the first hub 342.

Each of the first hub 342, the second hub 344, and the first and second rails 346 is disposed within the passageway 354 defined by the main body 340 of the actuator 326. The first hub 342 has a first end 358, a second end 360, and defines a second thread 362, a passageway 364, and first and second recesses 366. The second thread 362 extends from an exterior surface of the first hub 342 and mates with the first thread 356 defined by the main body 340 of the actuator 326. The passageway 364 extends from the first end 358 to the second end 360 of the first hub 342. Each of the first and second recesses 366 extends from the first end 358 to the second end 360 of the first hub 342 and is sized to receive a rail of the first and second rails 346, as described herein, to allow the first hub 342 to travel between first and second positions within the main body 340 of the actuator 326 during use. As the main body 340 of the actuator 326 is moved between its first and second positions, the threads 356, 362 interact with one another to achieve movement of the first hub 342 within the passageway 354 defined by the actuator 326. The first hub 342 is moveable along the first and second rails 346 between the first position and the second position. The first hub 342 is disposed at a first location when the actuator 326 is in the first position and at a second location, as shown in FIG. 14, when the actuator 326 is in the second position. In the first position, the first hub 342 is disposed a first distance from the distal end 322 of the housing 312. In the second position, the first hub 342 is disposed a second distance from the distal end 322 of the housing 312, which is different than the first distance. In the illustrated embodiment, the second distance is greater than the first distance.

The second hub 344 is disposed adjacent to, is attached to, and directly contacts the first hub 342 and is disposed between the first hub 342 and the housing 312. The second hub 344 has a first end 368, a second end 370, and defines a passageway 372, first and second recesses 374, a third recess 376, and a fourth recess 378. The passageway 372 extends from the first end 368 to the second end 370 of the second hub 344. Each of the first and second recesses 374 extends from the first end 368 to the second end 370 of the second hub 344 and is sized to receive a rail of the first and second rails 346, as described herein, to allow the second hub 44 to travel between first and second positions within the main body 340 of the actuator 326 during use. The third recess 376 extends from the second end 370 toward the first end 368 of the second hub 344, from an exterior surface of the second hub 344 toward the passageway 372 defined by the second hub 344, and is sized to receive a portion of the wire member 318, as described herein, to accomplish movement of the first elongate member 314 between its first, substantially straight configuration and its second, curved configuration. The fourth recess 378 is in communication with the third recess 376, extends from an exterior surface of the second hub 344 toward the passageway 372 defined by the second hub 344, and is sized to receive a portion of the wire member 18. The fourth recess 378 is sized to receive a portion of an attachment member (e.g., anchor, set screw) to accomplish attachment of the wire member 318 to the second hub 344. As the main body 340 of the actuator 326 is moved between its first and second positions, the threads 356, 362 interact with one another to achieve movement of the first hub 342 and the second hub 344 within the passageway 354 defined by the actuator 326. The second hub 344 is moveable along the first and second rails 346 between the first position and the second position. The second hub 344 is disposed at a first location when the actuator 326 is in the first position and at a second location, as shown in FIG. 14, when the actuator 326 is in the second position. In the first position, the second hub 344 is disposed a first distance from the distal end 322 of the housing 312. In the second position, the second hub 344 is disposed a second distance from the distal end 322 of the housing 312, which is different than the first distance. In the illustrated embodiment, the second distance is greater than the first distance.

One of the first and second rails 346 is disposed within a recess 366 defined by the first hub 342 and a recess 374 defined by the second hub 344. Each of the first and second rails 346 has a first end 380 and a second end 382. Each of the first and second rails 346 provides a track upon which the first and second hubs 342, 344 can move relative to the housing 312.

The locking mechanism 328 is adapted to releasably lock the first portion 312 in the housing first position and the housing second position. In the illustrated embodiment, the locking mechanism 322 comprises a spring activated main body 384 that has a first end 386, a second end 388, a depressible portion 390, a projection 392, and a spring 394. The main body 384 is moveable between first and second positions. The spring 394 biases the main body 384 to the first position, as shown in FIGS. 16 and 18. The projection 392 extends from the main body 384 and is sized to be disposed within one of the first and second notches 334 defined by the first portion 324 of the housing 312 such that the first portion 324 can be moved between the housing first position and the housing second position.

The first elongate member 314 is attached to the first portion 324 of the housing 312 and has a proximal end 402, a distal end 404, and a lumen 406. The first elongate member 314 is moveable relative to the second portion 325 of the housing 312 between a first elongate member first position when the first portion 324 is in the housing first position, as shown in FIG. 17, and a first elongate member second position when the first portion 324 is in the housing second position, as shown in FIG. 19. The first elongate member 314 is moveable between a first, substantially straight configuration when the actuator 326 is in the actuator first position and a second, curved configuration when the actuator 326 is in the actuator second position.

The second elongate member 316 is disposed through the second portion 325 of the housing 312, partially extends through the first portion 324 of the housing 312, and partially extends through the lumen 406 of the first elongate member 314. The second elongate member 316 has a proximal end 420, a distal end 422, and main body 424 that defines a lumen 426 sized to receive a portion of a secondary medical device (e.g., lead extraction device, lead, locking stylet). In the illustrated embodiment, the distal end 422 of the second elongate member 316 has a plurality of teeth 427 (e.g., plurality of sharp surfaces) and is disposed outside of the lumen 406 of the first elongate member 314 when the housing 212 is in the housing first position and the first elongate member 314 is in the first elongate member first position and the distal end 422 of the second elongate member 316 is entirely disposed within the lumen 406 of the first elongate member 314 when the housing 212 is in a housing second position and the first elongate member 314 is in the first elongate member second position. However, alternative embodiments can include a second elongate member that has a distal end that is partially disposed within a lumen of a first elongate member when a housing is in a housing second position and the first elongate member is in a first elongate member second position.

The wire member 318 has a first end 430 and a second end 432. The first end 430 of the wire member 318 is attached to the actuator 326 (e.g., the second hub 344 using a set screw) and the second end 432 is attached to the first elongate member 314 (e.g., the cap 412). The wire member 318 extends from the first end 430, through a portion of the actuator 326, through a portion of the first portion 324 of the housing 312, through the second portion 410 of the first elongate member 314, to the cap 412. The wire member 318 is disposed within the wall 414 of the second portion 410 of the first elongate member 314.

The capstan 319 is disposed on the post 333 of the second portion 325 of the housing 312 and provides a mechanism for allowing a user to wrap a secondary medical device (e.g., locking stylet, lead, locking stylet) around the capstan 319 such that the secondary medical device can be held in the same hand used to activate a trigger.

Various methods of using a deflectable catheter system are described herein. While the methods described herein are shown and described as a series of acts, it is to be understood and appreciated that the methods are not limited by the order of acts, as some acts may in accordance with these methods, occur in the order shown and/or described, in different orders, and/or concurrently with other acts described herein.

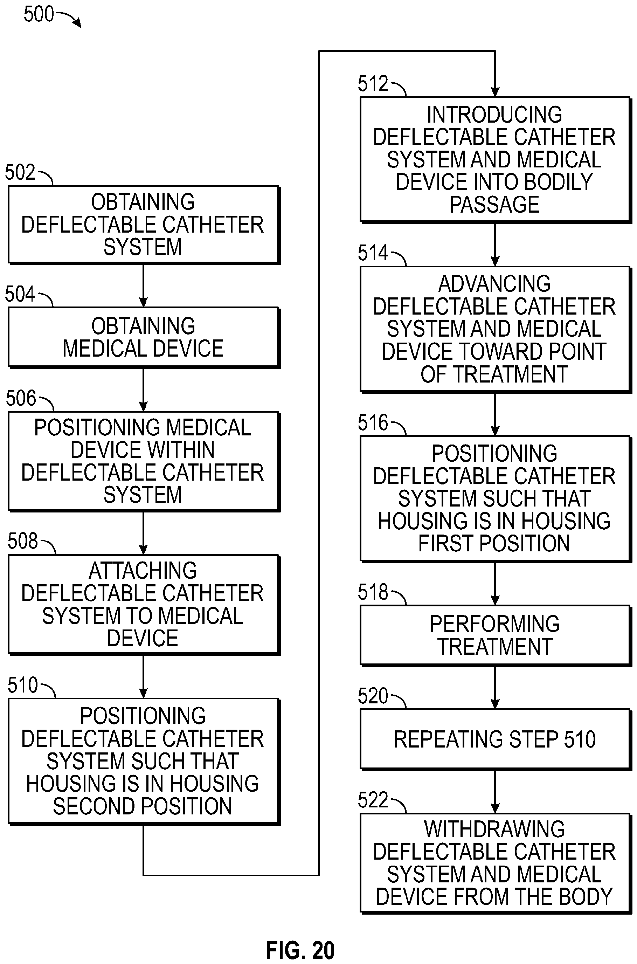

FIG. 20 illustrates a schematic illustration of an example method 500 of using a deflectable catheter system.

A step 502 comprises obtaining a deflectable catheter system that has a housing, a first elongate member, and a wire member. Another step 504 comprises obtaining a medical device that has a second elongate member. The second elongate member has a distal end. Another step 506 comprises positioning the second elongate member within the first elongate member of the deflectable catheter system. Another step 508 comprises attaching the deflectable catheter system to the medical device such that the housing of the deflectable catheter system is moveable between a housing first position and a housing second position. Another step 510 comprises positioning the deflectable catheter system such that the housing is in the housing second position and the distal end of the second elongate member is disposed (e.g., entirely) within the first elongate member of the deflectable catheter system. Another step 512 comprises introducing the deflectable catheter system and medical device into a bodily passage. Another step 514 comprises advancing the deflectable catheter system and medical device toward a point of treatment within the bodily passage. Another step 516 comprises positioning the deflectable catheter system such that the housing is in the housing first position and the distal end of the second elongate member is disposed outside of the first elongate member of the deflectable catheter system. Another step 518 comprises performing treatment. Another step 520 comprises repeating step 510. Another step 522 comprises withdrawing the deflectable catheter system from the bodily passage.

Step 502 can be accomplished using any suitable deflectable catheter system, such as those described herein. For example, the deflectable catheter system 10 illustrated in FIGS. 1, 2, 3, 4, 5, 6, 7, 8, 9, and 10 can be used.

Step 504 can be accomplished using any suitable medical device, such as those described herein. For example, the medical device 210 illustrated in FIGS. 11 and 12 can be used.

Step 506 can be accomplished by applying a force on the medical device directed toward the deflectable catheter system such that the second elongate member of the medical device is directed into, and passed through, the lumen of the first elongate member of the deflectable catheter system. Alternatively, step 502, step 504, and step 506 can comprise obtaining a deflectable catheter system, such as the deflectable catheter system 310 illustrated in FIGS. 13, 14, 15, 16, 17, 18, and 19.

Step 508 can be accomplished by applying a force on the deflectable catheter system directed toward the medical device until the deflectable catheter system becomes attached to the medical device (e.g., using a snap fit attachment). Alternatively, step 508 can be accomplished using one or more fasteners. Alternatively, when deflectable catheter 310 is being utilized, step 508 can be omitted from method 500.

Step 510 can be accomplished by applying a force on the locking mechanism and applying a force on the deflectable catheter system until it is moved into the housing second position. Alternatively, in embodiments in which deflectable catheter system 310 is being utilized, step 510 comprises positioning the deflectable catheter system such that the first portion of the housing is in the housing second position and the second elongate member is disposed (e.g., entirely) within the first elongate member. In embodiments in which deflectable catheter system 310 is being utilized, step 510 can be accomplished by applying a force on the locking mechanism and applying a force on the first portion of the housing until it is moved into the housing second position. Step 510 provides a mechanism to cover the distal end of the second elongate member to prevent damage to the bodily passage during use.

Step 512 can be accomplished by applying a distally-directed force on the deflectable catheter system and medical device toward the bodily passage. Alternatively, in embodiments in which deflectable catheter system 310 is being utilized, step 512 comprises introducing the deflectable catheter system into a bodily passage. In embodiments in which deflectable catheter system 310 is being utilized, step 512 can be accomplished by applying a distally-directed force on the deflectable catheter system toward the bodily passage.

Step 514 can be accomplished by applying a distally-directed force on the deflectable catheter system and medical device toward the point of treatment. Alternatively, in embodiments in which deflectable catheter system 310 is being utilized, step 514 comprises advancing the deflectable catheter system toward a point of treatment within the bodily passage. In embodiments in which deflectable catheter system 310 is being utilized, step 514 can be accomplished by applying a distally-directed force on the deflectable catheter system toward the point of treatment.

Step 516 can be accomplished by applying a force on the locking mechanism and applying a force on the deflectable catheter system until it is moved into the housing first position. Alternatively, in embodiments in which deflectable catheter system 310 is being utilized, step 516 comprises positioning the deflectable catheter system such that the first portion of the housing is in the housing first position and the second elongate member is disposed (e.g., entirely) outside the first elongate member. In embodiments in which deflectable catheter system 310 is being utilized, step 510 can be accomplished by applying a force on the locking mechanism and applying a force on the first portion of the housing until it is moved into the housing first position. Step 516 provides a mechanism to uncover the distal end of the second elongate member such that treatment can be performed.

Step 518 can be accomplished by activating one or more components (e.g., drive mechanism) disposed within the medical device or the second portion of the housing to perform treatment (e.g., activate rotation of the second elongate member by depressing the trigger and moving the trigger between its first and second positions). For example, optional steps comprise applying force on the trigger while maintaining the position of the handle; stopping the application of force on the trigger; and repeating the steps of applying force on the trigger and stopping the application of force on the trigger. Alternatively, step 518 can be accomplished by advancing one or more secondary medical devices (e.g., lead extraction device, lead, locking stylet) through the lumen defined by the second elongate member, performing treatment, and withdrawing the secondary medical device from the lumen and the bodily passage. Optionally, step 510, step 512, step 514, step 516, and/or step 518 can be repeated.

Optionally, step 520 can be omitted from method 500.

Step 522 can be accomplished by applying a proximally-directed force on the deflectable catheter system and medical device away from the bodily passage. Alternatively, in embodiments in which deflectable catheter system 310 is being utilized, step 522 comprises withdrawing the deflectable catheter system from the bodily passage. In embodiments in which deflectable catheter system 310 is being utilized, step 522 can be accomplished by applying a proximally-directed force on the deflectable catheter system away from the bodily passage.

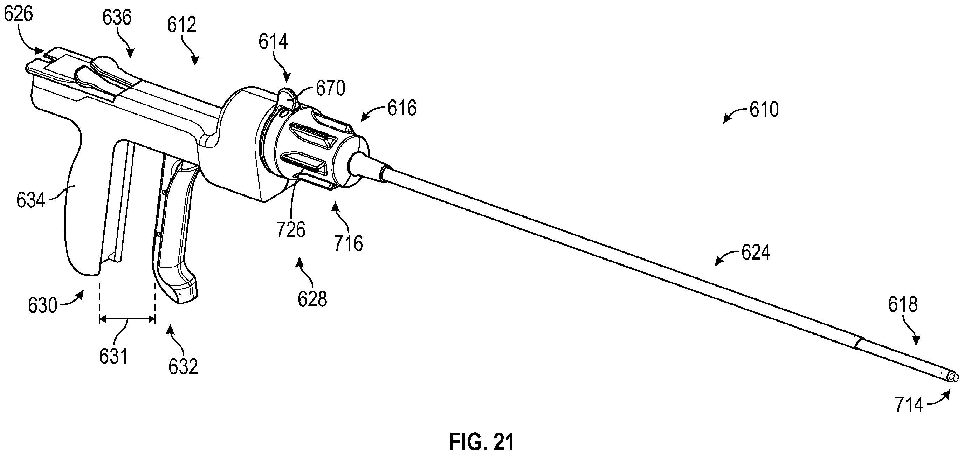

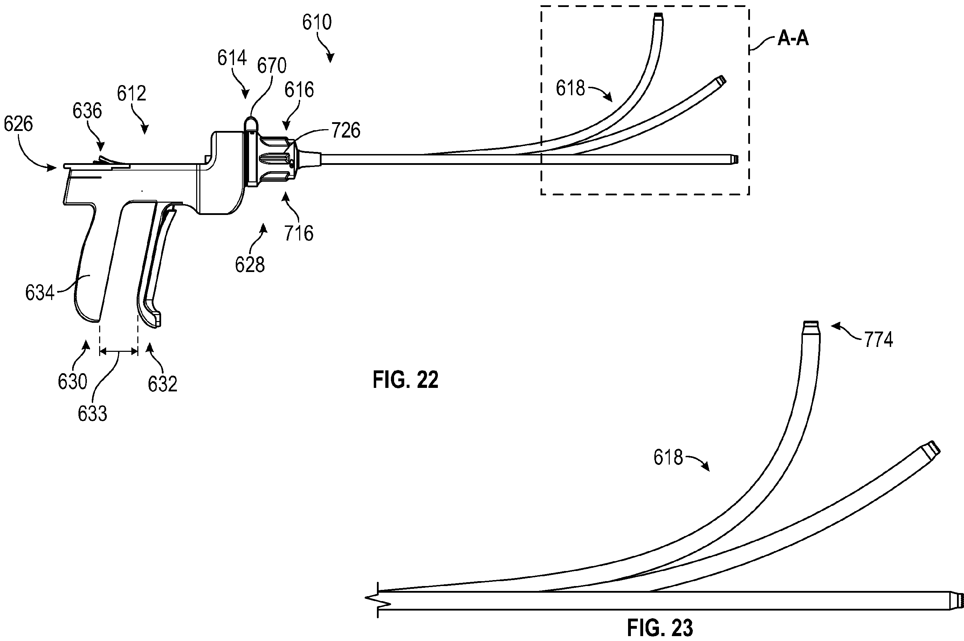

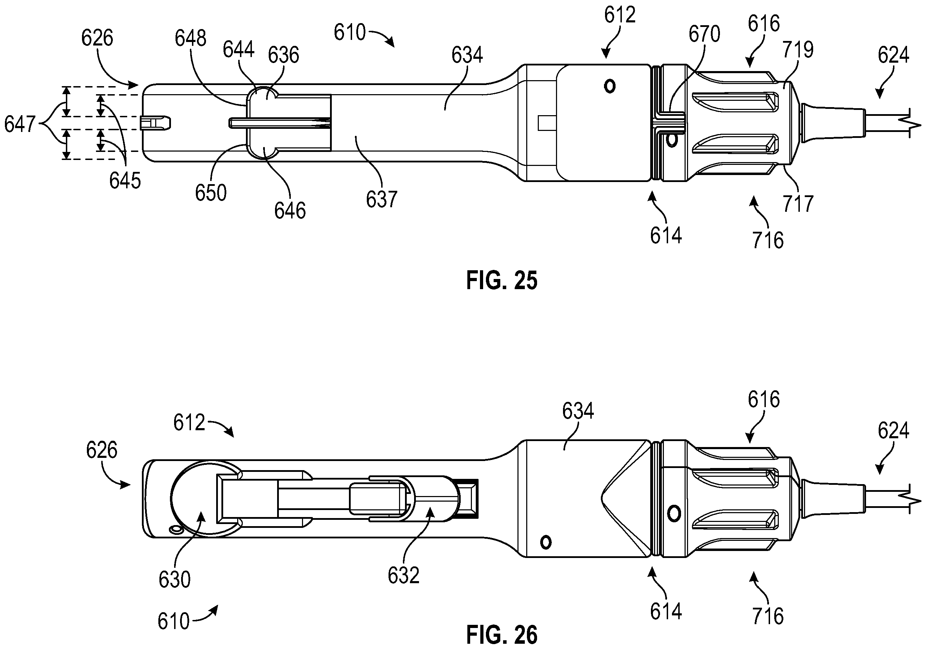

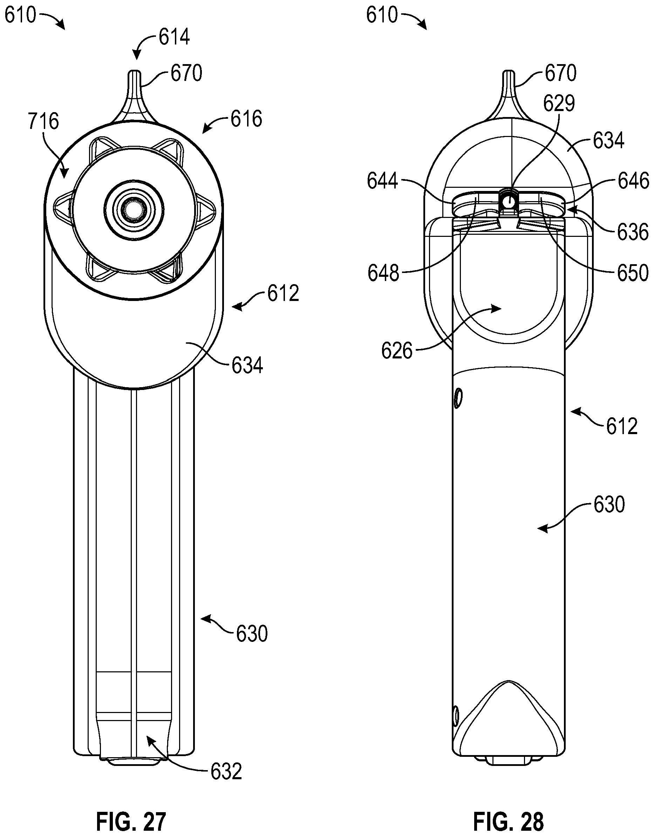

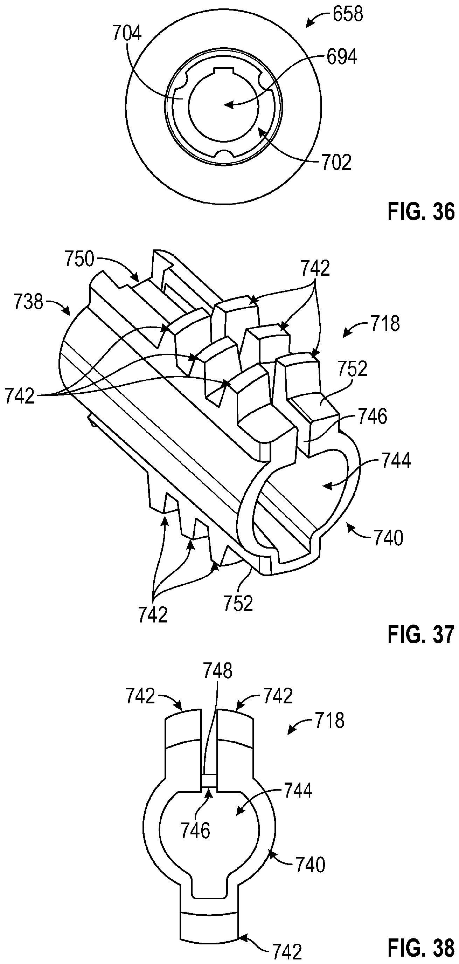

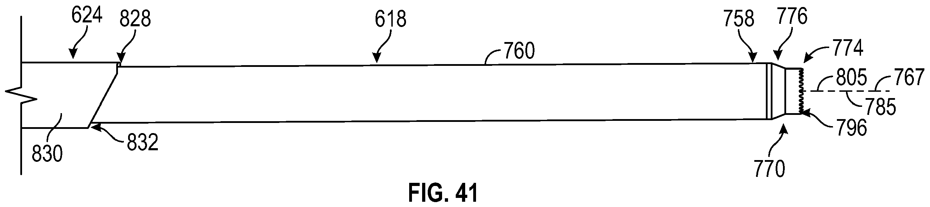

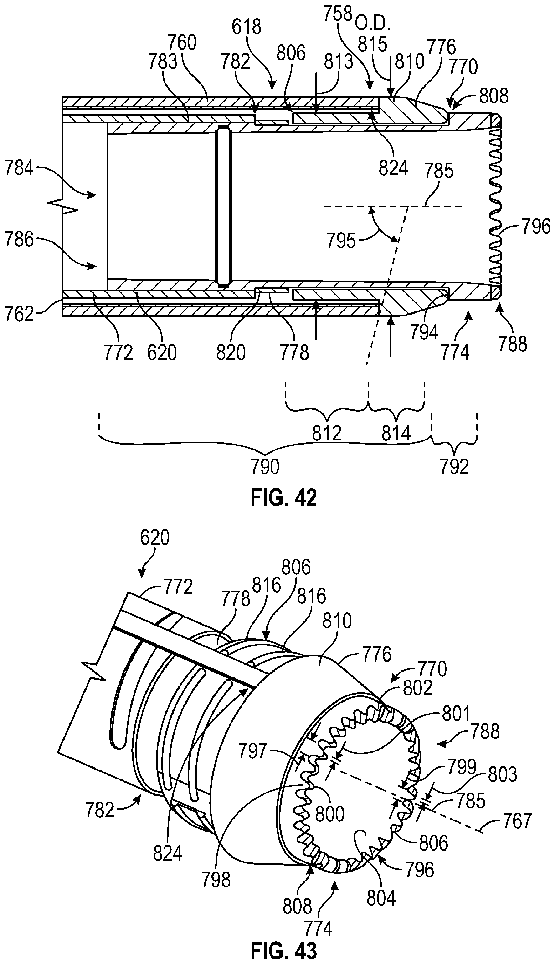

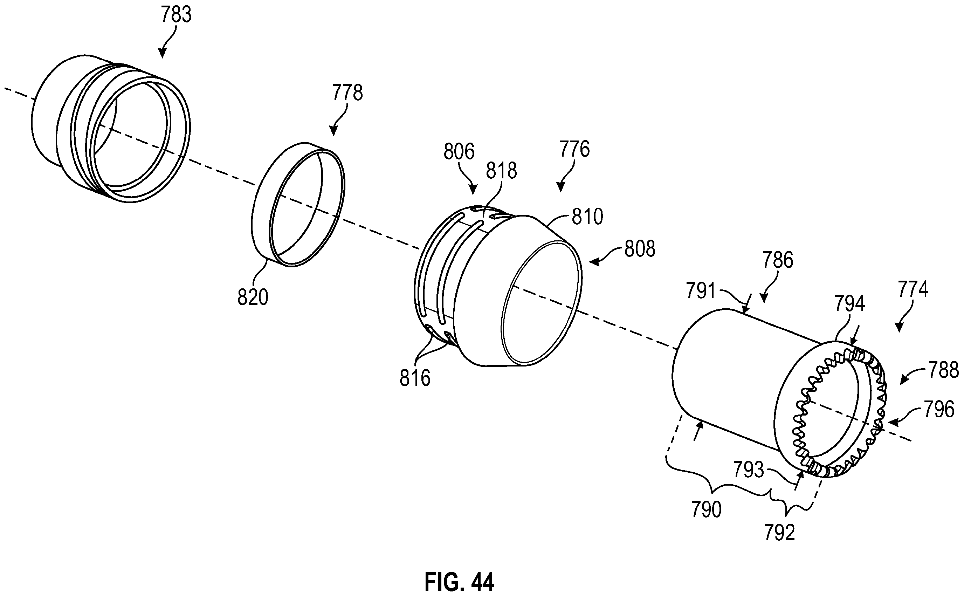

FIGS. 21 through 44 illustrate another example deflectable catheter system 610. The deflectable catheter system 610 is similar to the deflectable catheter system 310 illustrated in FIGS. 13, 14, 15, 16, 17, 18, and 19 and described above, except as detailed below. In the illustrated embodiment, the deflectable catheter system 610 has a housing 612, an orienting member 614, an actuator 616, a first elongate member 618, a second elongate member 620, a wire member 622, and a third elongate member 624. The deflectable catheter system 610 has a first, substantially straight configuration, as shown in FIGS. 21 and 41, and a second, curved configuration, as shown in FIGS. 22 and 23.

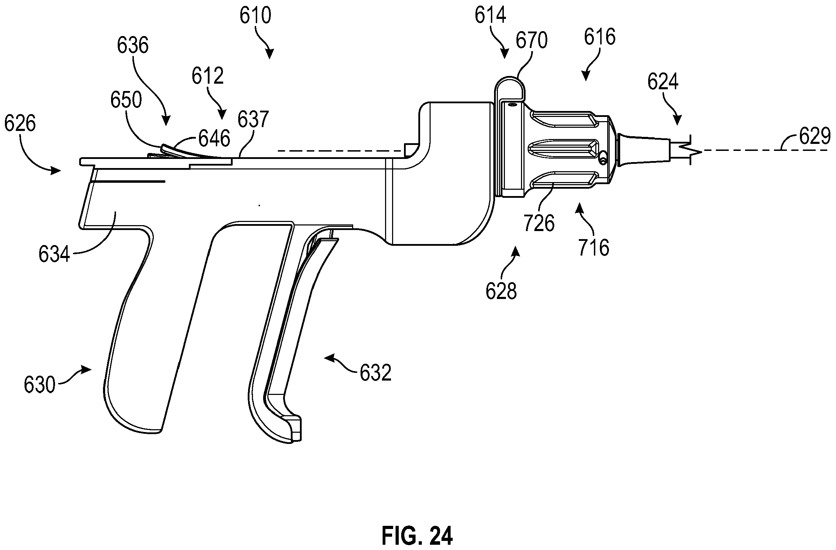

In the illustrated embodiment, the housing 612 has a proximal end 626, a distal end 628, a lengthwise axis 629, a handle 630, a trigger 632, and a main body 634 that defines an anchor member 636, a chamber 638, and a passageway 640 that extends through the chamber 638. The lengthwise axis 629 of the housing 612 extends through the passageway 640 defined by the housing 612. In the illustrated embodiment, the trigger 632 is moveable between a first position, as shown in FIGS. 21 and 24, in which the trigger 632 is disposed a first distance 631 from the handle 630 and a second position, as shown in FIG. 22, in which the trigger 632 is disposed a second distance 633 from the handle 630 that is less than the first distance 631. The trigger 632 is biased to the first position. A drive mechanism 642 housed within the housing 612 moves from an inactive state (e.g., first position) when the trigger 632 is in the first position and an active state (e.g., second position) when the trigger 632 is moved between the first and second positions resulting in rotation of the second elongate member 620 about the lengthwise axis 629 of the housing 612, as described in more detail herein. The second elongate member 620 is rotatable relative to the housing 612 and the first elongate member 618 when the drive mechanism 642 is in the active state. The trigger 632 can be moved from the first position to the second position by applying an axial force on the trigger 632 directed toward the handle 630 while maintaining the positon of the handle 630. The trigger 632 can be moved from the second position to the first position by removing any force being applied to the trigger 632.

Any suitable drive mechanism can be included in a deflectable catheter system and selection of a suitable drive mechanism can be based on various considerations, such as the intended use of the deflectable catheter system within which the drive mechanism is intended to be included. Examples of drive mechanisms considered suitable to include in a deflectable catheter system include conventional drive mechanisms for lead removal device, gear-pulley mechanisms, powered drive shafts, drive mechanisms capable of rotating a second elongate member, drive mechanisms capable of rotating a second elongate member in a first direction (e.g., when a trigger is advanced toward a handle) and rotating the second elongate member in a second direction (e.g., when a trigger is advanced away from a handle) such that the second elongate member is bidirectional, and any other drive mechanism considered suitable for a particular embodiment.

As shown in FIGS. 24 through 29, the anchor member 636 includes first and second posts 644, 646 that extend from an exterior surface 637 of the main body 634, away from the handle 630, and away from the distal end 628 of the housing 612 to an end 648, 650. Each of the first and second posts 644, 646 have a first width 645 at the exterior surface 637 of the housing 612 and a second width 647 at their respective ends 648, 650 that is greater than the first width 645. The anchor member 636 provides structure for a user to wrap a secondary medical device (e.g., lead extraction device, locking stylet, lead) around the anchor member 636 such that the secondary medical device can be held in the same hand used to activate the trigger 632.

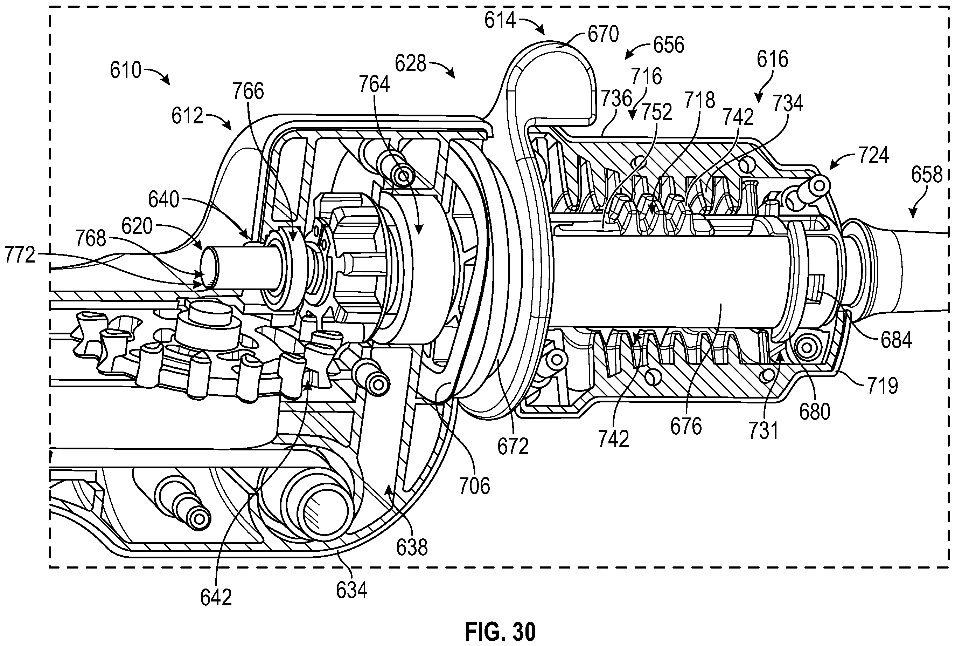

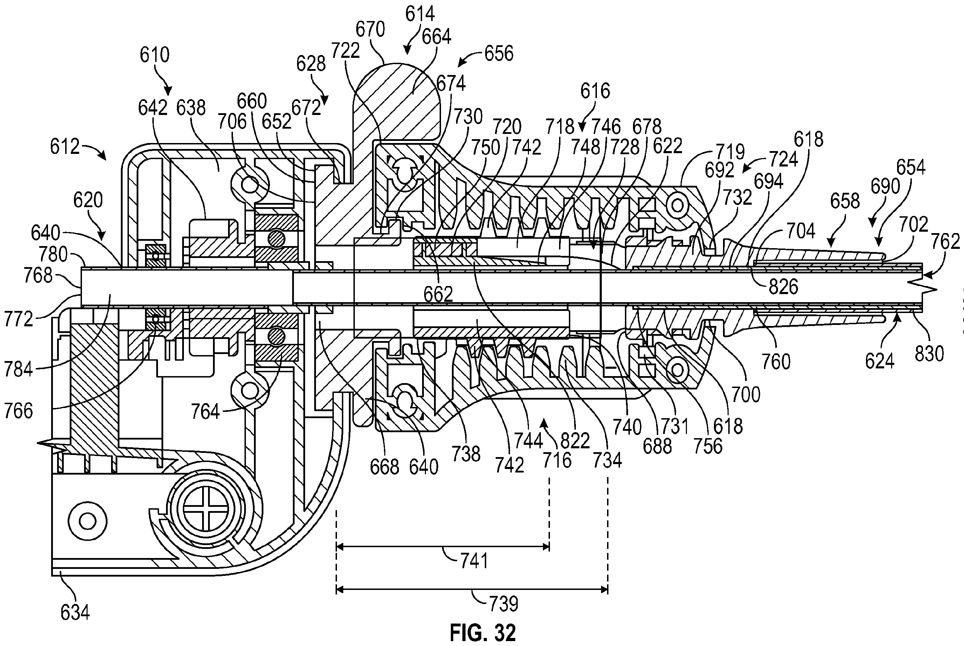

In the illustrated embodiment, and as shown in FIGS. 32, 34, and 35, the orienting member 614 is moveably attached to the housing 612 and has a lengthwise axis 651, a first end 652, a second end 654, a first portion 656, and a second portion 658. The first portion 656 has a first end 660, a second end 662, and a main body 664 that defines a base portion 666, a passageway 668, a lever 670, a first recess 672, a second recess 674, a first support arm 676, a second support arm 678, a first projection 680, a second projection 682, a first receiving passageway 684, and a second receiving passageway 686. The base portion 666 extends from the first end 660 and toward the second end 662. The passageway 668 extends from the first end 660 to the second end 662 and is sized to receive a portion of the second elongate member 620. The lever 670 extends away from the base portion 666, away from the lengthwise axis 651 of the orienting member 614, and is sized such that a user can apply torque on the lever 670, or a force on the level 670, while maintaining the position of the housing 612 to manipulate the position of the first elongate member 618, the wire member 622, and third elongate member 624 to direct, or orient, a curved defined by the deflectable catheter system 610 when in the second, curved configuration in a desired direction. For example, a curve defined by the first and second elongate members 618, 620 can be directed in any suitable direction since the orienting member 614 can be rotated around the entire circumference of the second elongate member 620. As shown in FIGS. 30 and 32, the orienting member 614 is partially disposed within the chamber 638 defined by the housing 612 and a portion of the housing 612 is disposed within the first recess 672. The first recess 672 is defined on the base portion 666 and between the lever 670 and the first end 660 of the first portion 656. The mating configuration between the main body 634 of the housing 612 and the main body 664 of the first portion 656 allows the orienting member 614 to rotate relative to the housing 612. The second recess 674 is defined on the base portion 666 and between the lever 670 and the second end 662 of the first portion 656. A portion of the actuator 616 is moveably disposed within the second recess 674. The mating configuration between the main body 716 of the actuator 616 and the main body 664 of the first portion 656 allows the actuator 616 to rotate relative to the orienting member 614 and the orienting member 614 to rotate relative to the actuator 616. Each of the first and second support arms 676, 678 extends from the base portion 666 and to the second end 662 of the first portion 656. The first projection 680 extends from the first support arm 676 and away from the lengthwise axis 651 of the orienting member 614 and is sized to be disposed within the second recess 731 defined by the actuator 616. The second projection 682 extends from the second support arm 678 and away from the lengthwise axis 651 of the orienting member 614 and is sized to be disposed within the second recess 731 defined by the actuator 616. The first receiving passageway 684 is disposed between the first projection 680 and the second end 662 and is sized to receive a portion of the second portion 658 of the orienting member 614. The second receiving passageway 686 is disposed between the second projection 682 and the second end 662 and is sized to receive a portion of the second portion 658 of the orienting member 614.

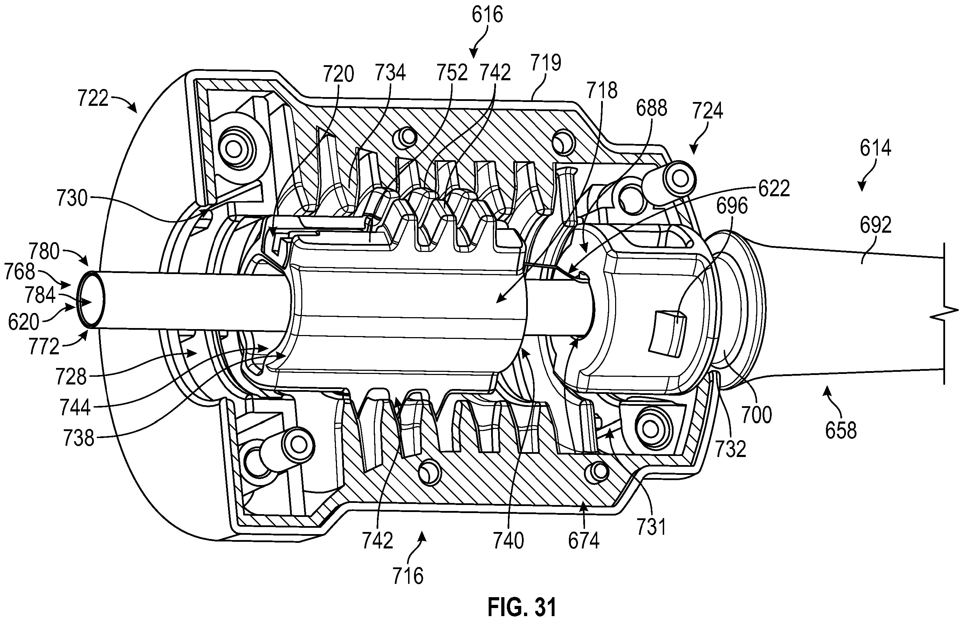

In the illustrated embodiment, and as shown in FIGS. 31, 32, and 35, the second portion 658 of the orienting member 614 is releasably attached to the first portion 656 of the orienting member 614 such that movement of the first portion 656 results in movement of the second portion 658. However, alternative embodiments can include a second portion that is permanently attached to a first portion or an orienting member that is formed as a single, contiguous piece of material. The second portion 658 has a first end 688, a second end 690, and a main body 692 that defines a passageway 694, a first projection 696, a second projection 698, a first recess 700, and a second recess 702. The passageway 694 extends from the first end 688 to the second end 690 and is sized to receive a portion of the first elongate member 618 and the second elongate member 620. The first projection 696 is disposed between the first end 688 and the first recess 700 and is sized to be received by the first receiving passageway 684 defined by the first portion 656 of the orienting member 614. The second projection 698 is disposed between the first end 688 and the first recess 700 and is sized to be received by the second receiving passageway 686 defined by the first portion 656 of the orienting member 614. The first recess 700 is defined between the first and second projections 696, 698 and the second end 690 and is sized to receive a portion of the actuator 616. In the illustrated embodiment, the retaining projection 732 of the main body 716 of the actuator 616 is moveably disposed within the first recess 700. The mating configuration between the main body 716 of the actuator 616 and the main body 692 of the second portion 658 allows the actuator 616 to rotate relative to the orienting member 614 and vice versa. The second recess 702 extends from the second end 690 toward the first end 688 within the passageway 694 to a recess base 704. The second recess 702 is sized to receive a portion of the third elongate member 624, which is partially disposed within the second recess 702. In alternative embodiments, an orienting member can be omitted from a deflectable catheter system and an actuator can be attached to a housing.

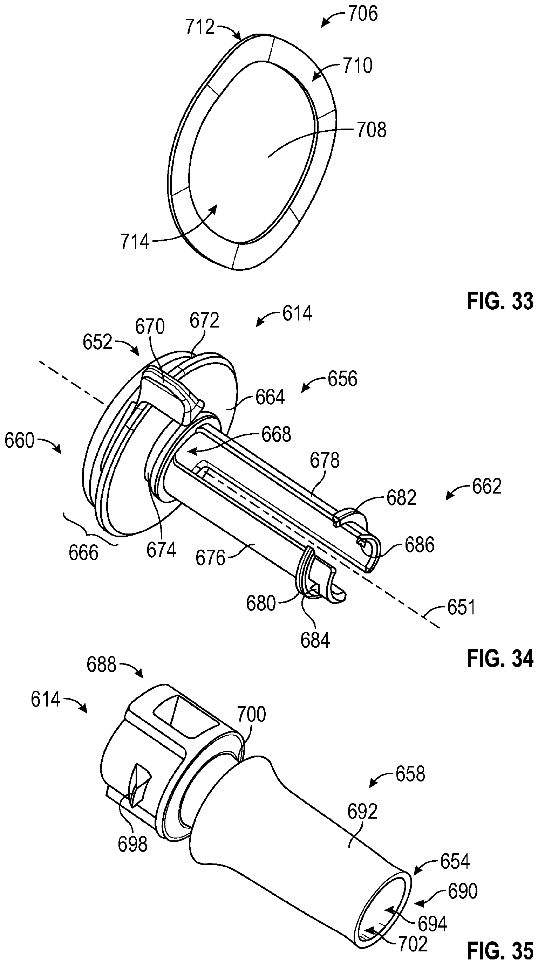

In the illustrated embodiment, the position of the orienting member 614 is maintained during use using a retaining member 706 that is disposed between the housing 612 and the orienting member 614. As shown in FIG. 33, the retaining member 706 has a center 708 and a main body 710 that defines a pre-defined undulating configuration 712 and a passageway 714. The pre-defined undulating configuration 712 provides a mechanism for maintaining the position of the orienting member 614 relative to the housing 612. The retaining member 706 is biased to the pre-defined undulating configuration 712 such that it partially contacts the housing 612 and the orienting member 614 and applies a force on both the housing 612 and the orienting member 706 to maintain the position of the orienting member 614 during use. In alternative embodiment in which an orienting member is omitted from a deflectable catheter system, a retaining member can be omitted or included such that it is disposed between a housing and an actuator to maintain the position of the actuator relative to the housing.

In the illustrated embodiment, the actuator 616 is disposed outside the chamber 638 defined by the main body 634 of the housing 612 and is moveably attached to the orienting member 614. The actuator 616 is attached to the housing 612 via the orienting member 614. However, in alternative embodiments (e.g., those that omit the inclusion of an orienting member), an actuator can be moveably attached (e.g., rotatably attached) to a housing (e.g., directly attached). The actuator 616 is moveable between an actuator first position, as shown in FIG. 21, and an actuator second position, as shown in FIGS. 22, 23, and 32, by applying torque on the actuator 626, or by applying a force on the actuator 626, while maintaining the position of the orienting member 614 and/or housing 612. Each of the first and second elongate members 618, 620 is disposed in a first, substantially straight configuration when the actuator 616 is in the actuator first position and each of the first and second elongate members 618, 620 is disposed in a second, curved configuration when the actuator 616 is in the actuator second position.

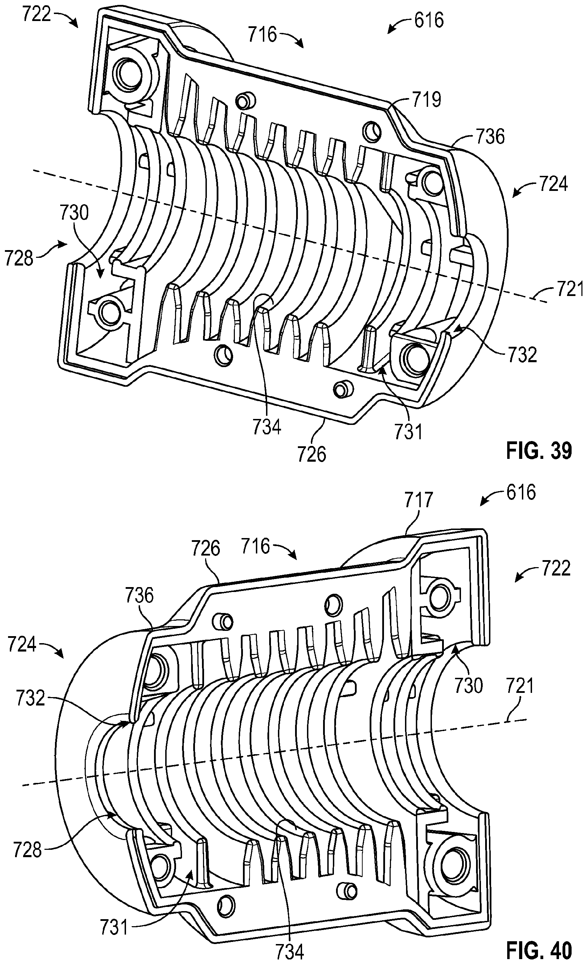

In the illustrated embodiment, the actuator 616 is rotatably attached to the first portion 656 of the orienting member 614 and the second portion 658 of the orienting member 614 and has a main body 716, a hub 718, and an attachment member 720. As shown in FIGS. 24, 30, 31, 32, 39, and 40, the main body 716 of the actuator 616 has a lengthwise axis 721, a first end 722, a second end 724, and defines a plurality of projections 726, a passageway 728, a first recess 730, a second recess 731, a retaining projection 732, and a first thread 734. In the illustrated embodiment, and as shown in FIGS. 39 and 40, the main body 716 of the actuator 616 is formed of a first portion 717 and a second portion 719 which are attached to another using any suitable technique or method of attachment (e.g., attachment members, adhesive). However, alternative embodiments can include a main body that is formed as a single, contiguous piece of material.

The plurality of projections 726 extend from an exterior surface 736 of the main body 716 and away from the lengthwise axis 721, provide a mechanism for a user to move the actuator 616 between the actuator first position and the actuator second position, and provide tactile feedback regarding the position of the main body 716. The passageway 728 extends from the first end 722 to the second end 724 and, in the illustrated embodiment, is coaxial with the passageway 640 defined by the housing 612. The passageway 728 is sized to receive a portion of the orienting member 614, the first elongate member 618, the second elongate member 620, the wire member 622, the hub 718, and the attachment member 720. The first recess 730 extends into the main body 716 within the passageway 728 and is sized to receive a portion of the orienting member 614. A portion of the orienting member 614 is moveably disposed within the recess 730. The mating configuration between the main body 664 of the first portion 656 of the orienting member 614 and the main body 716 of the actuator 616 allows the actuator 616 and orienting member 614 to rotate relative to the one another. The second recess 731 extends into the main body 716 within the passageway 728 and is sized to receive a portion of the orienting member 614. A portion of the orienting member 614 is moveably disposed within the second recess 731. The mating configuration between the main body 692 of the second portion 658 of the orienting member 614 and the main body 716 of the actuator 616 allows the actuator 616 and orienting member 614 to rotate relative to the one another. The retaining projection 732 is partially disposed within the first recess 700 defined by the second portion 658 of the orienting member 614. The first thread 734 extends into the passageway 728 and mates with the plurality of threaded portions 742 defined by the hub 718 such that movement of the main body 716 of the actuator 616 relative to the orienting member 614 results in movement of the hub 718.

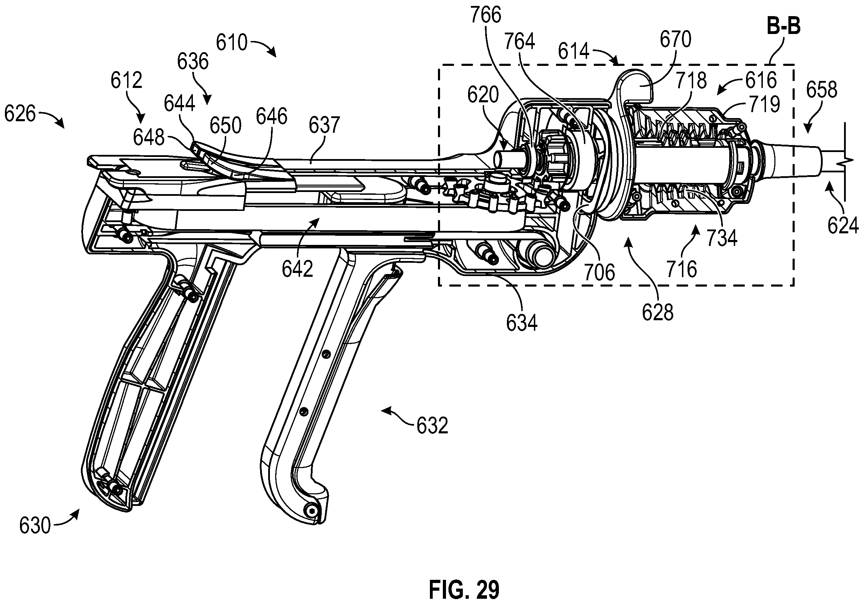

The hub 718 is disposed within the passageway 668 defined by the first portion 656 of the orienting member 614 between the first and second support arms 676, 678 and is disposed within passageway 728 defined by the main body 716 of the actuator 616. The hub 718 mates with the main body 716 to accomplish movement of the first and second elongate members 618, 620 between the first, substantially straight configuration and the second, curved configuration. As shown in FIGS. 29, 30, 31, 32, 37, and 38, the hub 718 has a first end 738, a second end 740, and defines a plurality of threaded portions 742, a passageway 744, a first recess 746, a ramped surface 748, and a second recess 750. Each threaded portion of the plurality of threaded portions 742 extends from an exterior surface 752 of the hub 718 and mates with the first thread 734 defined by the main body 716 of the actuator 616. The passageway 744 extends from the first end 738 to the second end 740 of the hub 718 and is sized to receive a portion of the second elongate member 620, which is partially disposed within the passageway 744. The first recess 746 extends from the second end 740 toward the first end 738 and is sized to receive a portion of the wire member 622. The ramped surface 748 is disposed within the first recess 746 and provides a mechanism for directing the wire member 622 into the second recess 750. The second recess 750 extends from the first end 738 toward the second end 740, is in communication with the first recess 746, and is sized to receive the attachment member 720. The attachment member 720 is disposed within the second recess 750 and is attached to the hub 718 using a friction fit between the two components. The wire member 622 is disposed between the hub 718 and the attachment member 720 and is held in place via the friction fit between the hub 718 and the attachment member 720.

As the main body 716 of the actuator 616 is moved between the actuator first position and the actuator second position, the first thread 734 and the plurality of threaded portions 742 interact with one another to achieve movement of the hub 718 within the passageway 728 defined by the main body 716 of the actuator 616. The hub 718 is moveable within the passageway 728 between a first position and a second position. The hub 718 is disposed at a first location when the actuator 616 is in the actuator first position and at a second location, as shown in FIG. 32, when the actuator 616 is in the actuator second position. In the first position, the hub 718 is disposed a first distance 739 from the distal end 628 of the housing 612. In the second position, the hub 718 is disposed a second distance 741 from the distal end 628 of the housing 612, which is different than the first distance 739. In the illustrated embodiment, the second distance 741 is less than the first distance 739.

The first elongate member 618 is attached to the orienting member 614. In the illustrated embodiment, the first elongate member 618 is partially disposed within the passageway 694 of the second portion 658 of the orienting member 614 and is attached to the second portion 658. The first elongate member 618 is attached to the orienting member 614 such that movement of the orienting member 614 results in movement of the first elongate member 618. For example, when torque, or a force, is applied to the lever 670 of the orienting member 614, the orienting member 614 and the first elongate member 618 rotate relative to the second elongate member 620. However, in embodiments in which an orienting member has been omitted, a first elongate member can be attached to a portion of an actuator.

As shown in FIGS. 32, 41, and 42, the first elongate member 618 and has a proximal end 756, a distal end 758, and a main body 760 that defines a lumen 762 through which the second elongate member 620 and the wire member 622 pass. In the illustrated embodiment, the proximal end 756 of the first elongate member 618 is attached to the orienting member 614 and the distal end 758 is attached to the second elongate member 620. The first elongate member 618 is attached to the ring member 776 of the second elongate member 620 via the plurality of projections 816 which provide a friction fit between the first elongate member 618 and the ring member 776. The first elongate member 618 is moveable between a first, substantially straight configuration when the actuator 616 is in the actuator first position and a second, curved configuration when the actuator 616 is in the actuator second position.

In the illustrated embodiment, the second elongate member 620 is disposed through the housing 612, extends through the passageway 640 defined by the housing 612, extends through the lumen 762 defined by the first elongate member 618, and is rotatably attached to the first elongate member 618. As shown in FIGS. 29, 30, and 32, the second elongate member 620 is rotatably attached to the housing 612 using a first bearing member 764 and a second bearing member 766. The second elongate member 620 is fixed axially relative to the housing 612. The second elongate member 620 is attached to the first bearing member 764 and the second bearing member 766. The first bearing member 764 is attached to the drive mechanism 642 housed within the housing 612 such that movement of the drive mechanism 642 results in rotation of the second elongate member 620 when the trigger 632 is moved between its first and second positions (e.g., the trigger 632 is depressed and released). While first and second bearings 764, 766 have been illustrated as accomplishing attachment between the second elongate member 620 and the housing 612, any suitable technique or method of attachment can be used between an elongate member and a housing that allows rotation of the elongate member relative to the housing.

As shown in FIGS. 29 through 32 and 41 through 43, the second elongate member 620 and has a lengthwise axis 767, a proximal end 768, a distal end 770, a tubular member 772, a distal tip 774, a ring member 776, and a retaining member 778. The second elongate member 620 is moveable between a first, substantially straight configuration when the actuator 616 is in the actuator first position and a second, curved configuration when the actuator 616 is in the actuator second position.

The tubular member 772 of the second elongate member 620 is attached to the first and second bearing members 764, 766, extends through the housing 612, extends through the passageway 668 defined by the first portion 656 of the orienting member 614, extends through the passageway 728 defined by the main body 716 of the actuator 616, extends through the passageway 744 defined by the hub 718, extends through the lumen 762 defined by the first elongate member 618, and is rotatable relative to the housing 612 and the first elongate member 618. The tubular member 772 of the second elongate member 620 has a proximal end 780, a distal end 782, and defines a lumen 784 sized to receive a portion of a secondary medical device (e.g., lead extraction device, lead, locking stylet). In the illustrated embodiment, the proximal end 780 of the tubular member 772 is disposed outside of the chamber 638 defined by the housing 612 and the distal end 782 is attached to the distal tip 774 and disposed within the lumen 762 defined by the first elongate member 618. In the illustrated embodiment, the tubular member 772 is attached to the ring member 776 using a connector 783. However, in alternative embodiments, a tubular member of a second elongate member can be attached, directly or otherwise, to a ring member without use of a connector.

The distal tip 774 is attached to the second elongate member 620 such that rotation of the second elongate member 620 results in rotation of the distal tip 774. The distal tip 774 is rotatable relative to the first elongate member 618. In the illustrated embodiment, the distal tip 744 has a lengthwise axis 785, a first end 786, a second end 788, a first portion 790, a second portion 792, an angled surface 794, and a plurality of teeth 796. The first portion 790 has a first outside diameter 791 and the second portion 792 has a second outside diameter 793 that is greater than the first outside diameter 791. The tubular member 772 of the second elongate member 620 is attached to the first portion 790 of the distal tip 774. The angled surface 794 extends from the first portion 790 to the second portion 792 and is disposed at an angle 795 relative to the lengthwise axis 785 that is less than 90 degrees. The structural arrangement between the angled surface 794 and the ring member 776 provides a mechanism for maintaining the axial position of the ring member 776 during use. The plurality of teeth 796 is disposed at the second end 788 of the distal tip 774 and provides a mechanism for dissecting tissue during use. Each tooth of the plurality of teeth 796 has a first end 798 and a second end 800. The first end 798 has a first width 797 on an exterior surface 802 of the distal tip 744 and a second width 799 on an interior surface 804 of the distal tip 744 that is less than the first width 797. The second end 800 has a third width 801 on the exterior surface 802 of the distal tip 744 and a fourth width 803 on the interior surface 804 of the distal tip 744 that is less than the third width 801. The third width 801 is less than the first width 797 and the fourth width 803 is less than the second width 799. Each tooth of the plurality of teeth 796 tapers from the exterior surface 802 to the interior surface 804 and from the first end 798 toward the second end 800. The second end 800 has a rounded surface 806 that extends from the exterior surface 802 to the interior surface 804.

In the illustrated embodiment, the ring member 776 is rotatably disposed on the distal tip 744 (e.g., first portion 790) between the retaining member 778 and the distal tip 744 (e.g., second portion 792). The ring member 776 is rotatable around the distal tip 744 when a force is applied to the orienting member 614, as described herein, and the second elongate member 620 (e.g., the tubular member 772) is rotatable relative to the first elongate member 618 and the ring member 776 when the trigger 632 is moved between its first and second positions. The ring member 776 has a lengthwise axis 805, a first end 807, a second end 808, and a main body 810 that defines a first portion 812, a second portion 814, a plurality of projections 816, and a recess 818. The second end 808 of the ring member 776 is tapered and provides a mechanism for advancing the deflectable catheter system 610 into a bodily passage while reducing, or preventing, damage to the tissue defining the bodily passage. The first portion 812 has a first outside diameter 813 and the second portion 814 has a second outside diameter 815 that is greater than the first outside diameter 813. The plurality of projections 816 is disposed on the first portion 812. The first elongate member 618 is attached to the first portion 812 of the ring member 776 using a friction fit between the plurality of projections 816 and the first elongate member 618. However, other techniques and/or methods of attachment can be utilized. The recess 818 extends from the first end 807 to the second portion 814 and is sized to receive the wire member 622, which is attached to the ring member 776.

In the illustrated embodiment, the retaining member 778 comprises a retaining ring 820 disposed between the second elongate member 620 and the ring member 776 and is attached (e.g., fixedly) to the distal tip 774. The retaining member 778 provides a mechanism for preventing axial movement of the ring member 776 during use while allowing rotation of the ring member 776 relative to the second elongate member 620 to direct any deflection of the first and second elongate members 618, 620 and allowing rotation of the second elongate member 620 relative to the first elongate member 618. While a retaining ring 820 has been illustrated, a retaining member 778 can comprise any suitable structural feature or component capable of retaining a ring member relative to a distal tip.

In the illustrated embodiment, and as shown in FIGS. 32, 42, and 43, the wire member 622 has a first end 822 and a second end 824. The first end 822 of the wire member 622 is attached to the actuator 616 and the second end 824 is attached to the ring member 776 and the first elongate member 618 (e.g., using adhesive). The first end 822 of the wire member 622 is attached within the second recess 750 defined by the hub 718 using the attachment member 720 and the second end 824 is attached within the recess 818 of the ring member 776 by welding the wire member 622 to the ring member 776. However, alternative techniques or methods of attachment can be utilized (e.g., adhesive). The wire member 622 extends from the first end 822, through a portion of the actuator 616 (e.g., first recess 746 of hub 718 and passageway 728 defined by main body 716), through a portion of the orienting member 614 (e.g., through a portion of the passageway 668 of the first portion 656 and through the passageway 694 of the second portion 658), between the first and second elongate members 618, 620 (e.g., within the lumen 762 defined by the first elongate member 618), and to the second end 824. In the illustrated embodiment, the wire member 622 is a flat wire member. However, alternative embodiments can include a wire member that has any suitable cross-sectional configuration, such as circular, rectangular, square, and any other cross-sectional configuration considered suitable for a particular embodiment. An example material considered suitable for a wire member includes super-elastic nitinol (e.g., 0.020").

In the illustrated embodiment, and as shown in FIGS. 32 and 41, the third elongate member 624 is slideably disposed on the first elongate member 618 and has a proximal end 826, a distal end 828, and a main body 830 that defines a lumen 832. The proximal end 826 is disposed within the second recess 702 defined by the second portion 658 of the orienting member 614 such that movement of the orienting member 614 results in movement of the third elongate member 624. However, alternative embodiments can include a third elongate member that does not move when an orienting member is moved. As shown in FIG. 41, the distal end 828 of the third elongate member 624 is tapered such that when the first and second elongate members 618, 620 are in the first, substantially straight configuration, the distal end 828 (e.g., terminal end) of the third elongate member 624 disposed on a plane that extends from the lengthwise axis 767 of the second elongate member 620 and contains the wire member 622. In the illustrated embodiment, the third elongate member 624 is moveable between a first, substantially straight configuration and a second, curved configuration with the first and second elongate members 618, 620. The third elongate member 624 is in the first, substantially straight configuration when the actuator 616 is in the actuator first position and the third elongate member 626 is in the second, curved configuration when the actuator 616 is in the actuator second position. However, alternative embodiments can omit the inclusion of a third elongate member, include a third elongate member that is oriented in any suitable manner relative to a wire member, and/or that has a distal end that is disposed between a curve defined by a first elongate member when in the second, curved configuration and a housing such that the third elongate member does not define a curve in the second, curved configuration.

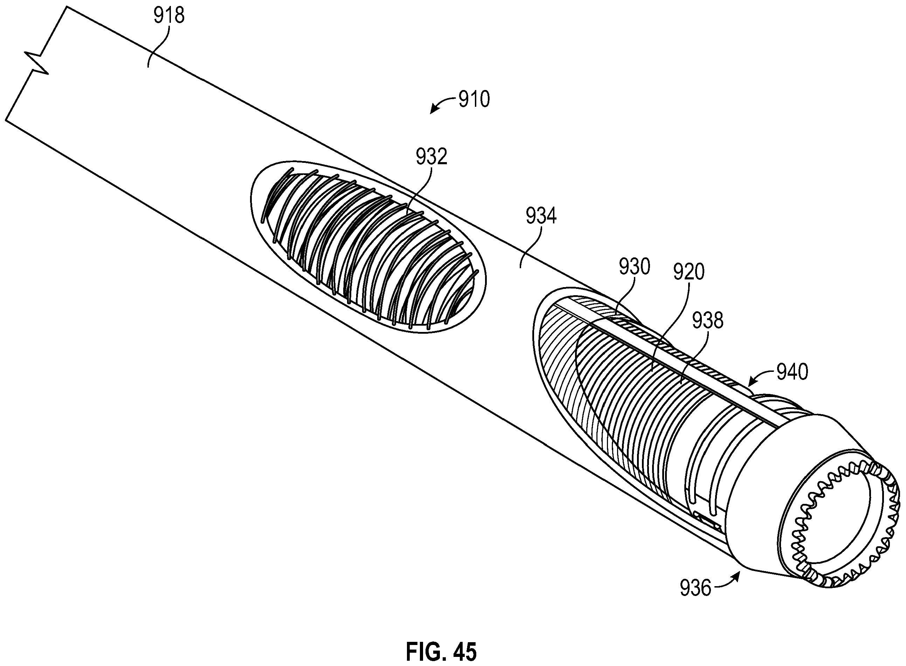

FIG. 45 illustrates the distal end of another example deflectable catheter system 910. The deflectable catheter system 910 is similar to the deflectable catheter system 610 illustrated in FIGS. 21 through 44 and described above, except as detailed below.

In the illustrated embodiment, the first elongate member 918 has an inner member 930 and an outer member 934. The inner member 930 is a tubular member formed of a first material and extends from the proximal end to the distal end 936 of the first elongate member 918. The outer member 934 is a tubular member formed of a second material and extends from the proximal end to the distal end 936 of the first elongate member 918. Alternative embodiments, however, can include an inner member and/or outer member that does not extend from a proximal end to a distal end of a first elongate member. The first material is different than the second material. The inner member 930 has a lubricious coating adjacent the second elongate member 920. The outer member 934 includes a braided material 932 disposed within the second material. In the illustrated embodiment, the second elongate member 920 is a coil member 938 formed of a plurality of flat wire members. Each flat wire member is formed of a third material and extends from the proximal end to the distal end 940 of the second elongate member 920. The third material is different than the first material of the first elongate member 918. Alternative embodiments, however, can include a second elongate member that includes only one coil member and/or such that the one coil member or plurality of coil members extend from a proximal end to a distal end of the second elongate member or that does/do not extend from a proximal end to a distal end of the second elongate member.

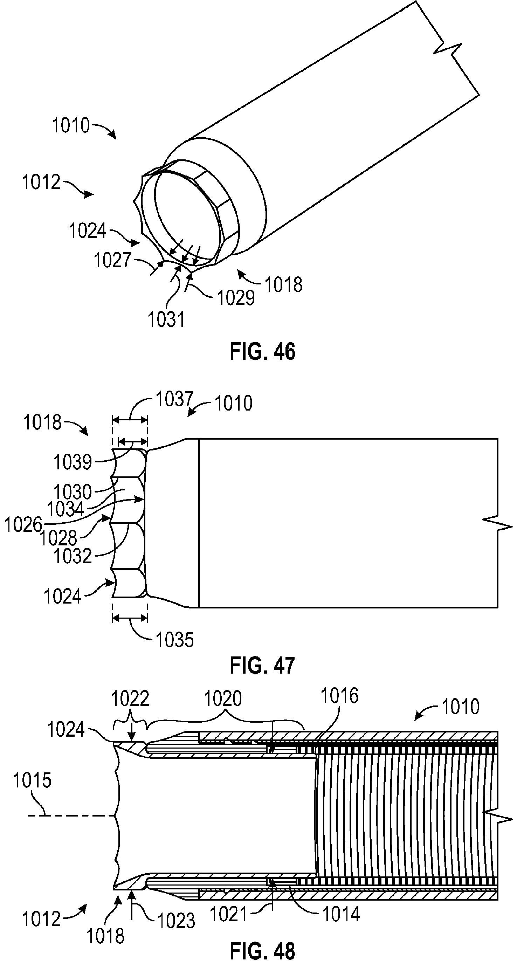

FIGS. 46, 47, and 48 illustrate the distal end of another example deflectable catheter system 1010. The deflectable catheter system 1010 is similar to the deflectable catheter system 610 illustrated in FIGS. 21 through 44 and described above, except as detailed below.

In the illustrated embodiment, the distal tip 1012 is attached to the second elongate member 1014 such that rotation of the second elongate member 1014 results in rotation of the distal tip 1012. The distal tip 1012 has a lengthwise axis 1015, a first end 1016, a second end 1018, a first portion 1020, a second portion 1022, and a plurality of teeth 1024. The first portion 1020 has a first outside diameter 1021 and the second portion 1022 has a second outside diameter 1023 that is greater than the first outside diameter 1021. The second elongate member 1014 is attached to the first portion 1020 of the distal tip 1012. The plurality of teeth 1024 is disposed at the second end 1018 of the distal tip 1012 and provides a mechanism for dissecting tissue during use. Each tooth of the plurality of teeth 1024 has a first end 1026, a second end 1028, a first side 1030, a second side 1032, and curved portion 1034 disposed between the first side 1030 and the second side 1032. Each tooth of the plurality of teeth 1024 has a first thickness 1027 at the first side 1030, a second thickness 1029 at the second side 1032, and a third thickness 1031 along the curved portion 1034 and between the first and second sides 1030, 1032. The third thickness 1031 is less than the first and second thicknesses 1027, 1029 such the thickness of each tooth of the plurality of teeth 1024 tapers from the first side 1030 toward the second side 1032 and tapers from the second side 1032 toward the first side 1030. Each tooth of the plurality of teeth 1024 has a first height 1035 at the first side 1030, a second height 1037 at the second side 1032, and a third height 1039 along the curved portion 1034 and between the first and second sides 1030, 1032. The third height 1039 is less than the first and second heights 1035, 1037 such the height of each tooth of the plurality of teeth 1024 tapers from the first side 1030 toward the second side 1032 and tapers from the second side 1032 toward the first side 1030.

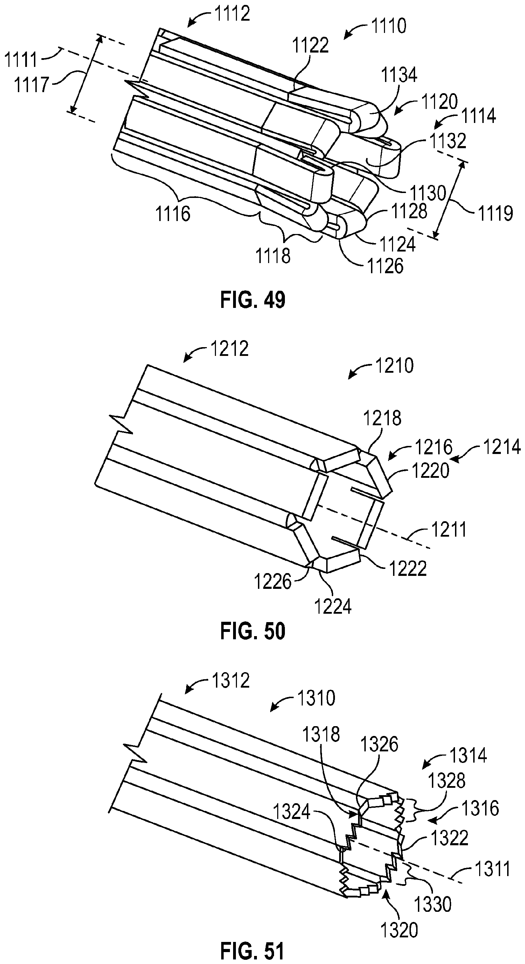

FIG. 49 illustrates another example distal tip 1110 that can be included in a deflectable catheter system. The distal tip 1110 is similar to the distal tip 774 illustrated in FIGS. 21, 22, 23, and 41 through 44 and described above, except as detailed below. The distal tip 1110 has a lengthwise axis 1111, a first end 1112, a second end 1114, a first portion 1116, a second portion 1118, and a plurality of teeth 1120. The first portion 1116 has a first inside diameter 1117 and the second portion 1118 has a second inside diameter 1119 that is greater than the first inside diameter 1117 such that the distal tip 1110 flares outwardly and away from the lengthwise axis 1111 from the first portion 1116 to the second end 1114. The plurality of teeth 1120 is disposed at the second end 1114 of the distal tip 1110 and provides a mechanism for dissecting tissue during use. Each tooth of the plurality of teeth 1120 has a first end 1122, a second end 1124, a first side 1126, a second side 1128, and is separated by an adjacent tooth of the plurality of teeth 1120 by a gap 1130. In the illustrated embodiment, each tooth of the plurality of teeth 1120 comprises a piece of material 1132 folded over on itself to form a curved surface 1134 on the second end 1114. Each of the first and second sides 1126, 1128 provides a sharp surface to dissect tissue while the curved surface 1134 prevents damage to tissue while the deflectable catheter system is being advanced into, or retracted within, a bodily passage. A tooth can have any suitable length extending from the first side 1126 to the second side (e.g., 1 millimeter, 2 millimeters). A distal tip can be formed using any suitable material, such as those described herein (e.g., medical grade stainless steel, folded sheet metal) and can be manufactured using any suitable technique or method, such as using a CNC machine, photo etched, water jet, and/or laser cut.

FIG. 50 illustrates another example distal tip 1210 that can be included in a deflectable catheter system. The distal tip 1210 is similar to the distal tip 774 illustrated in FIGS. 21, 22, 23, and 41 through 44 and described above, except as detailed below. The distal tip 1210 has a lengthwise axis 1211, a first end 1212, a second end 1214, and a plurality of teeth 1216. The plurality of teeth 1216 is disposed at the second end 1214 of the distal tip 1210 and provides a mechanism for dissecting tissue during use. Each tooth of the plurality of teeth 1216 has a first end 1218, a second end 1220, a first side 1222, a second side 1224, and is separated by an adjacent tooth of the plurality of teeth 1216 by a gap 1226. Each of the first and second sides 1222, 1224 provides a sharp surface to dissect tissue. In the illustrated embodiment, the second end 1224 of each tooth of the plurality of teeth 1216 is planar and is disposed on a plane that is perpendicular to the lengthwise axis 1211. In alternative embodiments, each tooth of a plurality of teeth can include radiused and/or contoured edges.

FIG. 51 illustrates another example distal tip 1310 that can be included in a deflectable catheter system. The distal tip 1310 is similar to the distal tip 774 illustrated in FIGS. 21, 22, 23, and 41 through 44 and described above, except as detailed below. The distal tip 1310 has a lengthwise axis 1311, a first end 1312, a second end 1314, and a plurality of teeth 1316. The plurality of teeth 1316 is disposed at the second end 1314 of the distal tip 1310, provides a mechanism for dissecting tissue during use, and includes about 54 threads per inch. Each tooth of the plurality of teeth 1316 has a first end 1318, a second end 1320, a first side 1322, a second side 1324, and is separated by an adjacent tooth of the plurality of teeth 1316 by a gap 1326. Each tooth of the plurality of teeth 1316 tapers from the second end 1320 toward the first end 1318 such that the first side 1322 is disposed at a first angle relative to the portion 1328 of the distal tip 1310 disposed within the gap 1326 and the second side 1324 is disposed at a second angle relative to the portion 1328 of the distal tip 1310 disposed within the gap 1326. The second angle is different than the first angle. The first angle is greater than 90 degrees and the second angle is about 90 degrees. Each tooth can have any suitable pitch, such as pitches between 0.4 millimeters and 0.6 millimeters. Each of the first and second sides 1322, 1324 provides a sharp surface to dissect tissue. A first set of teeth 1328 is oriented in a first direction around the circumference of the distal tip 1310 and a second set of teeth 1330 is oriented in a second direction around the circumference of the distal tip 1310 that is different than the first direction such that the orientation of the plurality of teeth 1316 alternates around the circumference of the distal tip 1310. This allows the distal tip 1310 to dissect tissue bi-directionally.

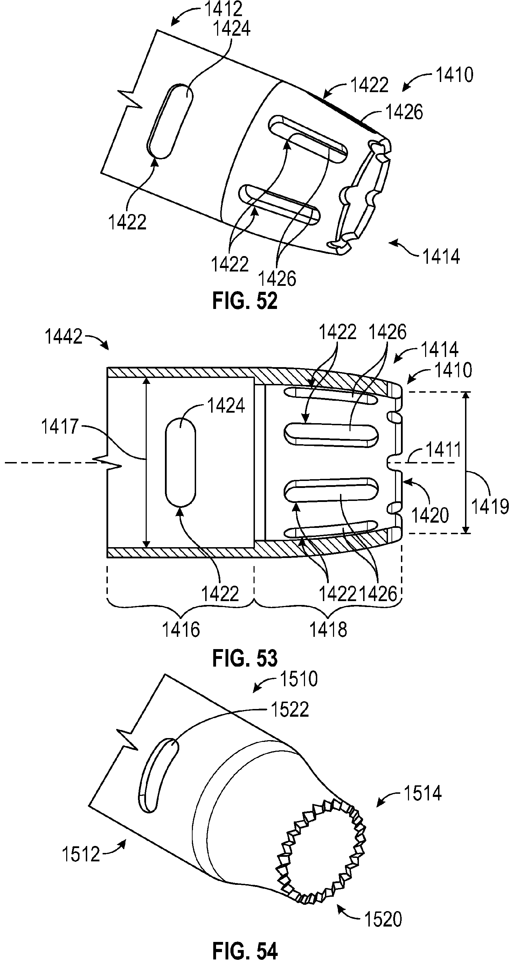

FIGS. 52 and 53 illustrate another example distal tip 1410 that can be included in a deflectable catheter system. The distal tip 1410 is similar to the distal tip 774 illustrated in FIGS. 21, 22, 23, and 41 through 44 and described above, except as detailed below. The distal tip 1410 has a lengthwise axis 1411, a first end 1412, a second end 1414, a first portion 1416, a second portion 1418, a plurality of teeth 1420, and a plurality of windows 1422. The first portion 1416 extends from the first end 1412 toward the second end 1414 and the second portion 1418 extends from the second end 1414 to the first portion 1416. The first portion 1416 has a first inside diameter 1417 and the second portion 1418 has a second inside diameter 1419 that is less than the first inside diameter 1417. The plurality of teeth 1420 is disposed at the second end 1414 of the distal tip 1410 and provides a mechanism for dissecting tissue during use. A first set of windows 1424 of the plurality of windows 1422 is disposed on the first portion 1416 and a second set of windows 1426 of the plurality of windows 1422 is disposed on the second portion 1418. Each window of the plurality of windows 1422 has a sharp edge along its entire perimeter to remove material (e.g., tissue) during use. Alternative embodiments, however, can include a window, or plurality of windows, that does not have a sharp edge or that has a sharp edge along a portion of its perimeter. A window included in a distal tip can have any suitable length (e.g., 3.2 millimeters), width (e.g., 0.6 millimeters), and include a notch between each tooth of a plurality of teeth that has any suitable width (e.g., 0.5 millimeters) and depth (e.g., 0.5 millimeters).

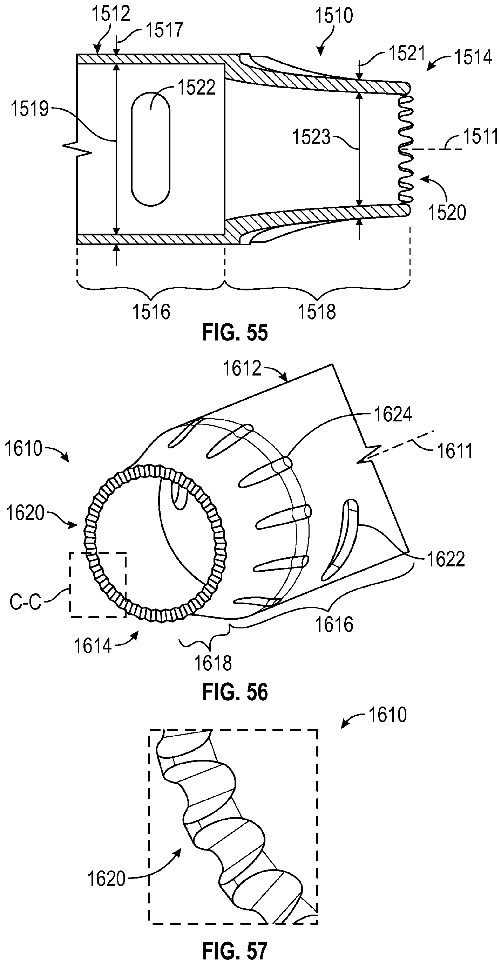

FIGS. 54 and 55 illustrate another example distal tip 1510 that can be included in a deflectable catheter system. The distal tip 1510 is similar to the distal tip 774 illustrated in FIGS. 21, 22, 23, and 41 through 44 and described above, except as detailed below. The distal tip 1510 has a lengthwise axis 1511, a first end 1512, a second end 1514, a first portion 1516, a second portion 1518, a plurality of teeth 1520, and a plurality of windows 1522. The first portion 1516 extends from the first end 1512 toward the second end 1514 and the second portion 1518 extends from the second end 1514 to the first portion 1516. The first portion 1516 has a first outside diameter 1517 and a first inside diameter 1519. The second portion 1518 has a second outside diameter 1521 that is less than the first outside diameter 1517 and a second inside diameter 1523 that is less than the first inside diameter 1519. The second inside diameter 1523 increases from the second end 1514 toward the first end 1512 (e.g., a distance of between about 0.7 millimeters and about 1 millimeter). The plurality of teeth 1520 is disposed at the second end 1514 of the distal tip 1510, provides a mechanism for dissecting tissue during use, and can have any suitable pitch (e.g., 0.5 millimeters). Each window of the plurality of windows 1522 is disposed on the first portion 1516.

FIGS. 56 and 57 illustrate another example distal tip 1610 that can be included in a deflectable catheter system. The distal tip 1610 is similar to the distal tip 1510 illustrated in FIGS. 54 and 55 and described above, except as detailed below. In the illustrated embodiment, the distal tip 1610 has a lengthwise axis 1611, a first end 1612, a second end 1614, a first portion 1616, a second portion 1618, a plurality of teeth 1620, a plurality of windows 1622, and a plurality of notches 1624. Each notch of the plurality of notches 1624 is partially disposed on the first portion 1616 and the second portion 1518 and provides a mechanism for dissecting tissue across the widest part of the distal tip 1610 during use.

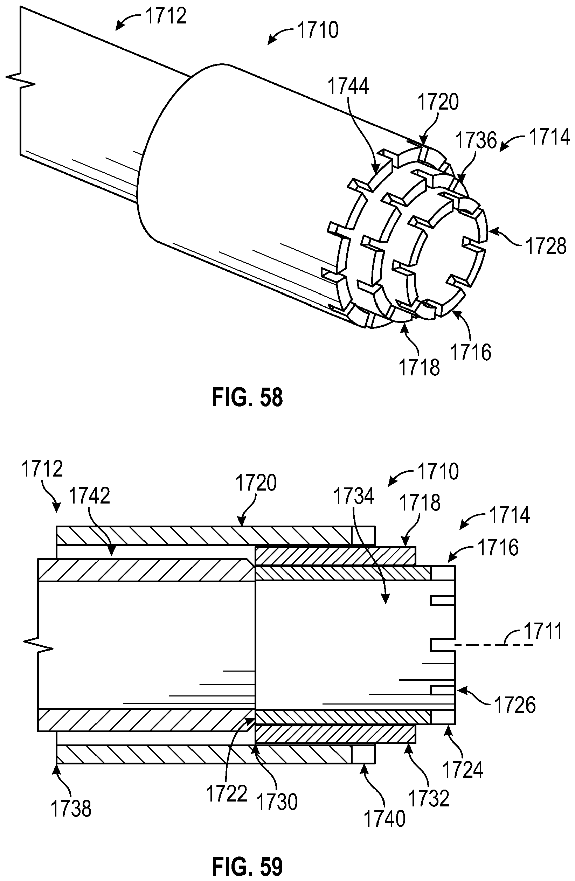

FIGS. 58 and 59 illustrates another example distal tip 1710 that can be included in a deflectable catheter system. The distal tip 1710 is similar to the distal tip 774 illustrated in FIGS. 21, 22, 23, and 41 through 44 and described above, except as detailed below. The distal tip 1710 has a lengthwise axis 1711, a first end 1712, a second end 1714, a first tubular member 1716, a second tubular member 1718, and a third tubular member 1720. The first tubular member 1716 has a first end 1722, a second end 1724, and defines a lumen 1726 and a plurality of teeth 1728. The second tubular member 1718 has a first end 1730, a second end 1732, and defines a lumen 1734 and a plurality of teeth 1736. The third tubular member 1720 has a first end 1738, a second end 1740, and defines a lumen 1742 and a plurality of teeth 1744. The first tubular member 1716 is partially disposed within the lumen 1734 defined by the second tubular member 1718 and is partially disposed outside of the lumen 1734 defined by the second tubular member 1718. The second tubular member 1718 is partially disposed within the lumen 1742 defined by the third tubular member 1720 and is partially disposed outside of the lumen 1742 defined by the third tubular member 1720. Each tooth of a plurality of teeth included in distal tip 1710 is separated from an adjacent tooth by a slot that can have any suitable width (e.g., 0.3 millimeters) and any suitable depth (e.g., 1 millimeter).

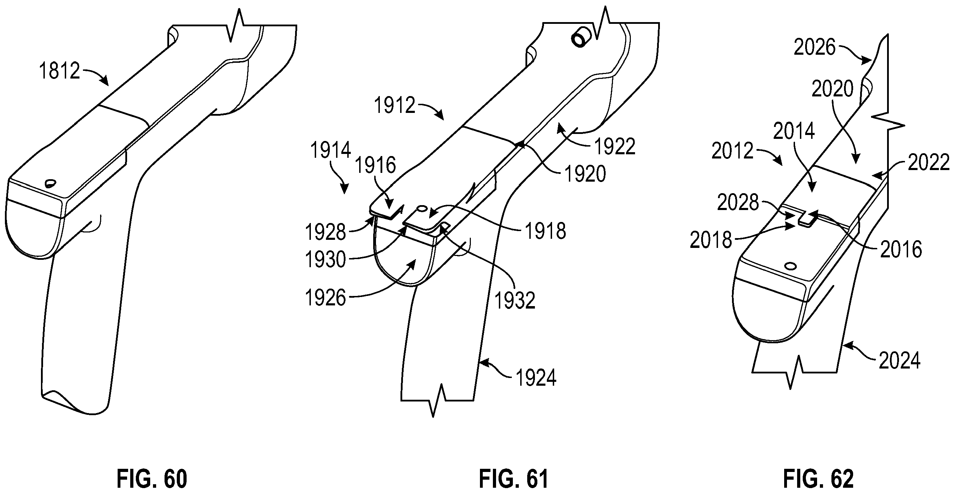

FIG. 60 illustrates another example housing 1812 that can be included in a deflectable catheter system. The housing 1812 is similar to the housing 612 illustrated in FIGS. 21, 22, 24 through 30, and 32 and described above, except as detailed below. In the illustrated embodiment, the housing 1812 omits the inclusion of an anchor member, such as anchor member 636 illustrated in FIGS. 21, 22, 24, 25, 28, and 29.

FIG. 61 illustrates another example housing 1912 that can be included in a deflectable catheter system. The housing 1912 is similar to the housing 612 illustrated in FIGS. 21, 22, 24 through 30, and 32 and described above, except as detailed below. In the illustrated embodiment, the housing 1912 includes an alternative anchor member 1914 that includes first and second posts 1916, 1918 that extend from an exterior surface 1920 of the main body 1922 of the housing 1912 and away from the distal end 1926 of the housing 1912 to ends 1928, 1930. Each of the first and second posts 1916, 1918 has a constant width that extends from the housing 1912 to the respective ends 1928, 1930. In addition, the housing 1912 defines a recess 1932 that extends into the main body 1922 between the posts 1916, 1918 and a distal portion of the housing 1912 that is sized to receive a portion of a secondary medical device (e.g., locking stylet, lead, lead removal device). The anchor member 1914 provides a mechanism for allowing a user to wrap a secondary medical device (e.g., locking stylet, lead, lead removal device) around the anchor member 1914 such that the secondary medical device can be held in the same hand used to activate a trigger.

FIG. 62 illustrates another example housing 2012 that can be included in a deflectable catheter system. The housing 2012 is similar to the housing 612 illustrated in FIGS. 21, 22, 24 through 30, and 32 and described above, except as detailed below. In the illustrated embodiment, the housing 2012 includes an alternative anchor member 2014 that includes first post 2016 and a recess 2018. The first post 2016 extends from an exterior surface 2020 of the main body 2022 of the housing 2012, away from the handle 2024, and away from the proximal end 2026 of the housing 2012 to an end 2028. The recess 2018 is defined by the main body 2022 of the housing 2012 such that the first post 2016 extends over the recess 2018. The anchor member 2014 provides a mechanism for allowing a user to wrap a secondary medical device (e.g., locking stylet, lead, lead removal device) around the anchor member 2014 such that the secondary medical device can be held in the same hand used to activate a trigger.

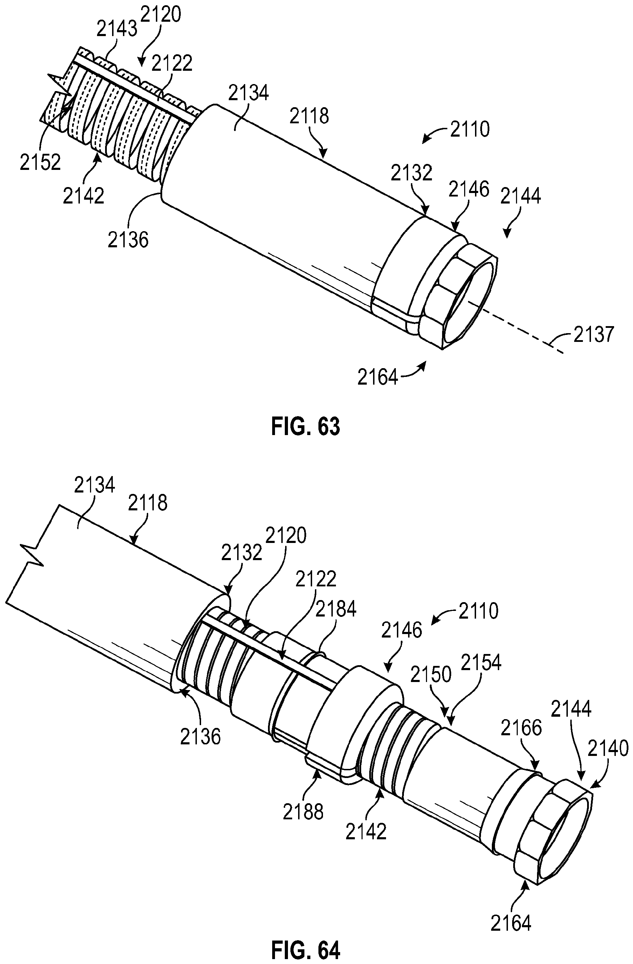

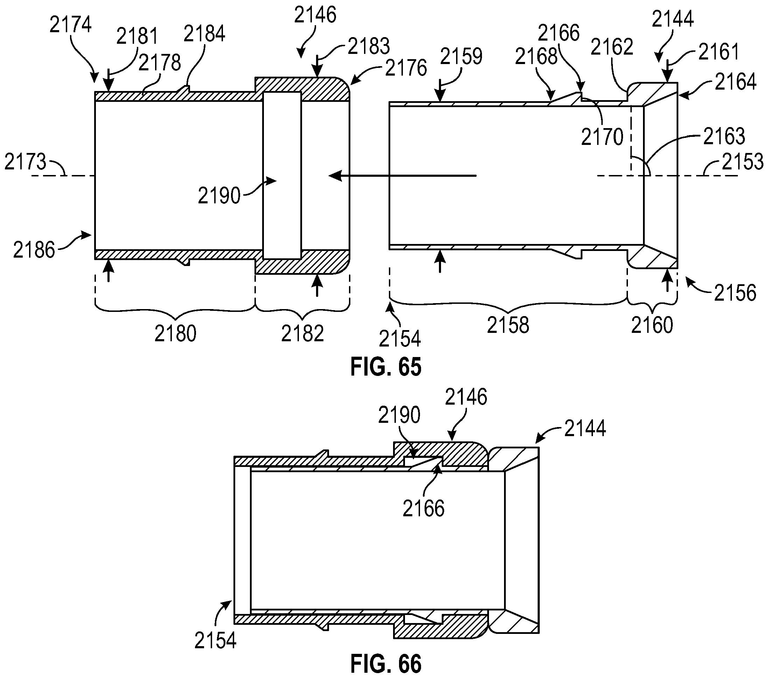

FIGS. 63, 64, 65, and 66 illustrate the distal end of another example deflectable catheter system 2110. The deflectable catheter system 2110 is similar to the deflectable catheter system 610 illustrated in FIGS. 21 through 44 and described above, except as detailed below.

In the illustrated embodiment, the first elongate member 2118 has a distal end 2132 and a main body 2134 that defines a lumen 2136 through which the second elongate member 2120 and the wire member 2122 are disposed.

In the illustrated embodiment, the second elongate member 2120 has a lengthwise axis 2137, a distal end 2140, a tubular member 2142, a distal tip 2144, and a ring member 2146. The tubular member 2142 of the second elongate member 2120 extends through the lumen 2136 defined by the first elongate member 2118. The tubular member 2142 of the second elongate member 2120 has a distal end 2150, and defines a lumen 2152 sized to receive a portion of a secondary medical device (e.g., lead extraction device, lead, locking stylet). In the illustrated embodiment, the tubular member 2142 is a coil member 2143 and the distal end 2150 is attached to the distal tip 2144 and disposed within the lumen 2136 defined by the first elongate member 2118.

The distal tip 2144 is attached to the tubular member 2142 such that rotation of the tubular member 2142 results in rotation of the distal tip 2144. In the illustrated embodiment, the distal tip 2144 has a lengthwise axis 2153, a first end 2154, a second end 2156, a first portion 2158, a second portion 2160, an angled surface 2162, a plurality of teeth 2164, and a projection 2166. The first portion 2158 has a first outside diameter 2159 and the second portion 2160 has a second outside diameter 2161 that is greater than the first outside diameter 2159. The tubular member 2142 of the second elongate member 2120 is attached to the first portion 2158 of the distal tip 2144. The angled surface 2162 extends from the first portion 2158 to the second portion 2160 and is disposed at an angle 2163 relative to the lengthwise axis 2153 that is equal to about 90 degrees. The plurality of teeth 2164 is disposed at the second end 2156 of the distal tip 2144 and provide a mechanism for dissecting tissue during use. The projection 2166 is disposed on the first portion 2158 and has a first end 2168 and a second end 2170. The height of the projection 2166 tapers from the second end 2170 toward the first end 2168.

In the illustrated embodiment, the ring member 2146 is rotatably attached to the distal tip 2144. The ring member 2146 is rotatable around the first portion 2158 of the distal tip 2144 when a force is applied to an orienting member and the second elongate member 2120 is rotatable relative to the first elongate member 2118 (e.g., the ring member 2146) when a trigger is moved between its first and second positions. The ring member 2146 has a lengthwise axis 2173, a first end 2174, a second end 2176, and a main body 2178 that defines a first portion 2180, a second portion 2182, a projection 2184, a lumen 2186, a slot 2188, and a recess 2190. The second end 2176 of the ring member 2146 is rounded and provides a mechanism for advancing the deflectable catheter system 2110 into a bodily passage while reducing, or preventing, damage to tissue defining the bodily passage. The first portion 2180 has a first outside diameter 2181 and the second portion 2182 has a second outside diameter 2183 that is greater than the first outside diameter 2181. The projection 2184 is disposed on the first portion 2180. The first elongate member 2118 is attached to the first portion 2180 of the ring member 2146 using the projection 2184 (e.g., via friction fit, welding, and/or adhesives). The lumen 2186 extends from the first end 2174 to the second end 2176 and is sized to receive a portion of the distal tip 2144 and a secondary medical device. The slot 2188 extends from the second end 2176 toward the first end 2174 to the projection 2184. The slot 2188 provides a mechanism for attaching the ring member 2146 to the distal tip 2144 by allowing the inside diameter of the lumen 2186 to expand and contract as the second end 2176 is passed over the projection 2166 defined by the distal tip 2144. The recess 2190 extends away from the lengthwise axis 2173 within the lumen 2186 and extends circumferentially around the main body 2178. The projection 2166 defined by the distal tip 2144 is partially disposed within the recess 2190.

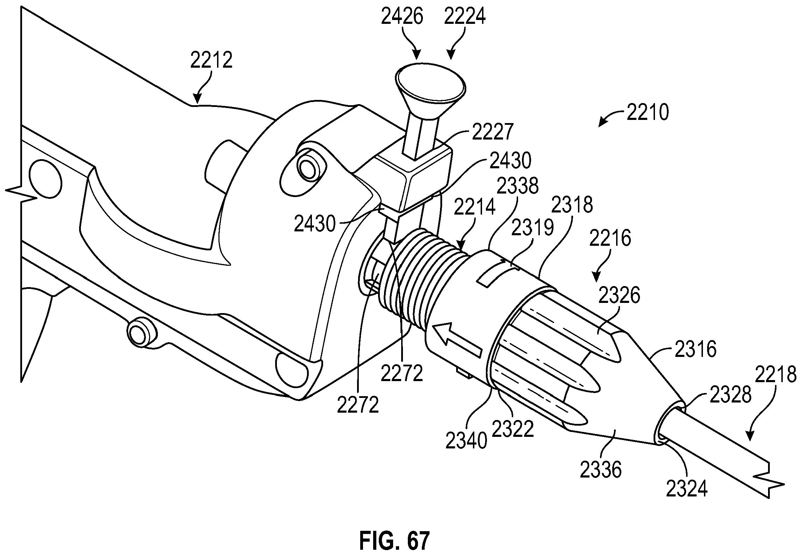

FIGS. 67, 68, 69, and 70 illustrate another example deflectable catheter system 2210. The deflectable catheter system 2210 is similar to the deflectable catheter system 610 illustrated in FIGS. 21 through 44 and described above, except as detailed below. In the illustrated embodiment, the deflectable catheter system 2210 has a housing 2212, an orienting member 2214, an actuator 2216, a first elongate member 2218, a second elongate member 2220, a wire member 2222, and a locking member 2224. The deflectable catheter system 2210 has a first, substantially straight configuration and a second, curved configuration.

In the illustrated embodiment, the orienting member 2214 is moveably attached to the housing 2212 and has a lengthwise axis 2251, first end 2252, a second end 2254, and a main body 2264 that defines a base portion 2266, a passageway 2268, a slot 2270, a plurality of locking member recesses 2272, a first elongate member recess 2274, and a first thread 2276. The base portion 2266 extends from the first end 2252 and toward the second end 2254 and is disposed within the chamber 2238 defined by the housing 2212. The passageway 2268 extends from the first end 2252 to the second end 2254 and is sized to receive a portion of the second elongate member 2220. The slot 2270 extends from the second end 2254 toward the first end 2252 and is sized to receive a portion of the actuator 2216, as described in more detail herein. Each locking member recess of the plurality of locking member recesses 2272 is disposed between the housing 2212 and the first thread 2276 and is sized to receive a portion of the locking member 2224. The first elongate member recess 2274 extends from the second end 2254 toward the first end 2252 and is sized to receive a portion of the first elongate member 2218. The first thread 2276 extends away from the lengthwise axis 2251 and mates with the second thread 2234 defined by the actuator 2216 to accomplish deflection of the first and second elongate members 2216, 2218.

In the illustrated embodiment, the actuator 2216 is disposed outside the chamber 2238 defined by the main body 2234 of the housing 2212 and is moveably attached to the orienting member 2214. The actuator 2216 is moveable between an actuator first position and an actuator second position by applying torque on the actuator 2216 while maintaining the position of the orienting member 2214 (e.g., using the locking member 2224). Each of the first and second elongate members 2218, 2220 is disposed in a first, substantially straight configuration when the actuator 2216 is in the actuator first position and each of the first and second elongate members 2218, 2220 is disposed in a second, curved configuration when the actuator 2216 is in the actuator second position.

In the illustrated embodiment, the actuator 2216 is rotatably attached to the orienting member 2214 and has a main body 2316, a hub 2318, and an attachment member 2320. The main body 2316 of the actuator 2216 has a lengthwise axis 2321, a first end 2322, a second end 2324, and defines a plurality of recesses 2326, a passageway 2328, and a second thread 2334. The plurality of recesses 2326 extend toward the lengthwise axis 2321 from an exterior surface 2336 of the main body 2316, provide a mechanism for a user to move the actuator 2216 between the actuator first position and the actuator second position, and provide tactile feedback regarding the position of the main body 2316. The passageway 2328 extends from the first end 2322 to the second end 2324 and, in the illustrated embodiment, is coaxial with the passageway 2240 defined by the housing 2212. The passageway 2328 is sized to receive a portion of the orienting member 2214, the first elongate member 2218, the second elongate member 2220, the wire member 2222, and the attachment member 2220. The second thread 2334 extends into the passageway 2328 and mates with the first thread 2276 defined by the orienting member 2214 such that movement of the main body 2316 of the actuator 2216 relative to the orienting member 2214 results in movement of the hub 2318.

The hub 2318 is disposed over a portion of the orienting member 2214 and between the locking member 2224 and the main body 2316 of the actuator 2216. The hub 2318 has a first end 2338, a second end 2340, and defines a first passageway 2344, a projection 2346, a second passageway 2348, and a recess 2350. The first passageway 2344 extends from the first end 2338 to the second end 2340 of the hub 2318 and is sized to receive a portion of the orienting member 2214 and the second elongate member 2220, which is partially disposed within the passageway 2344. The projection 2346 extends into the first passageway 2344 and is disposed within the slot 2270 defined by the orienting member 2214. The second passageway 2348 extends from the first end 2338 to the second end 2340 and through the projection 2346, is in communication with the recess 2350, and is sized to receive a portion of the wire member 2222. The recess 2350 extends from an exterior surface 2351 of the hub 2318, toward the first passageway 2344, and is in communication with the second passageway 2348. The attachment member 2320 is disposed within the recess 2350, comprises a set screw, and is attached to the hub 2318. The wire member 2222, as described in more detail herein, is disposed between the hub 2318 and the attachment member 2320 and is held in place via the attachment member 2320. The second end 2340 of the hub 2318 contacts the first end 2322 of the main body 2316 to accomplish movement of the first and second elongate members 2218, 2220 between the first, substantially straight configuration and the second, curved configuration. Alternatively, a user can apply a proximally-directed force on the hub 2318 while maintaining the position of the housing 2212 to accomplish movement of the first and second elongate members 2218, 2220 from the first, substantially straight configuration to the second, curved configuration and can release the proximally-directed force being applied to the hub 2318 while maintaining the position of the housing 2212 to accomplish movement of the first and second elongate members 2218, 2220 from the second, curved configuration to the first, substantially straight configuration.

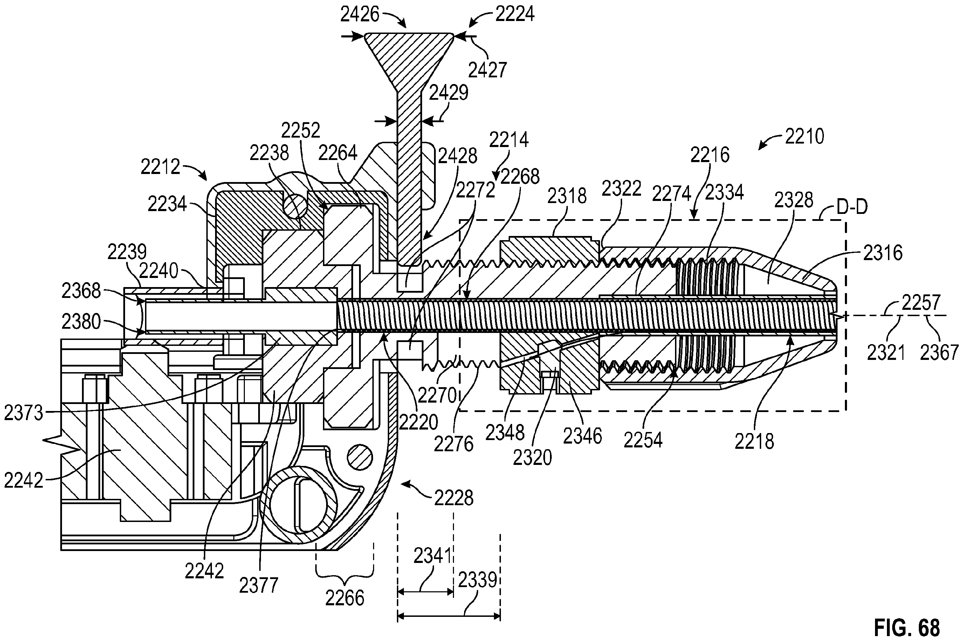

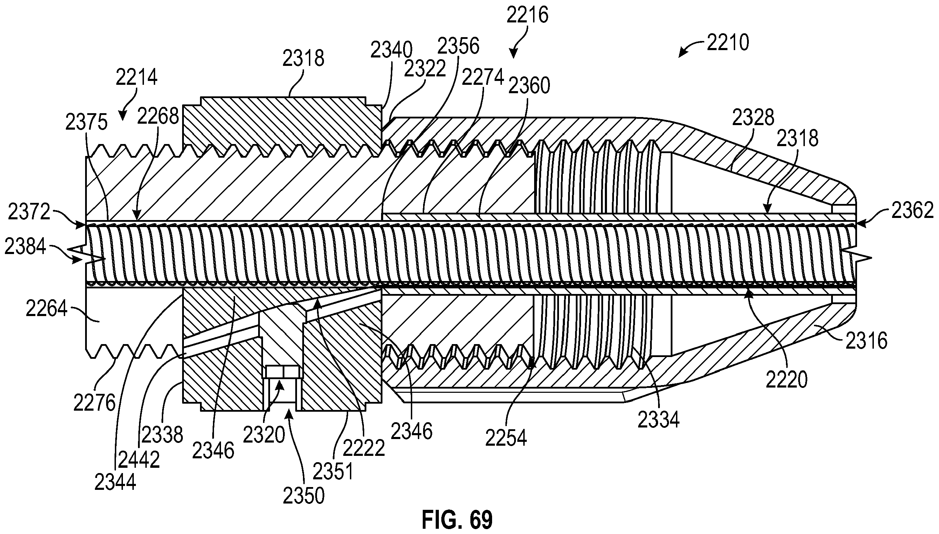

As the main body 2316 of the actuator 2216 is moved between the actuator first position, as shown in FIGS. 68 and 69, and the actuator second position, the first thread 2276 of the orienting member 2214 and the second thread 2334 of the main body 2316 interact with one another to achieve movement of the hub 2318 over the orienting member 2214. The hub 2318 is moveable over the orienting member 2214 between a first position, as shown in FIGS. 68 and 69, and a second position. The hub 2318 is disposed at a first location when the actuator 2216 is in the actuator first position and at a second location when the actuator 2216 is in the actuator second position. In the first position, the hub 2318 is disposed a first distance 2339 from the distal end 2228 of the housing 2212. In the second position, the hub 2318 is disposed a second distance 2341 from the distal end 2228 of the housing 2212, which is different than the first distance 2339. In the illustrated embodiment, the second distance 2341 is less than the first distance 2339.

In use, and while the locking member 2224 is in the first position, a user can apply torque to the hub 2318 while maintaining the position of the housing 2212 to manipulate the position of the orienting member 2214, the first elongate member 2218, and the wire member 2222 to direct a curved defined by the deflectable catheter system 2210 when in the second, curved configuration in a desired direction. Arrows 2319 defined on hub 2318 illustrated the direction which the first and second elongate members 2218, 2220 will deflect when the actuator is moved to the actuator second position.

The first elongate member 2218 is attached to the orienting member 2214. The first elongate member 2218 is attached to the orienting member 2214 such that movement of the hub 2318 and the orienting member 2214 results in movement of the first elongate member 2218. For example, when torque is applied to the hub 2318, the orienting member 2214, the first elongate member 2218, and the wire member 2222 rotate relative to the second elongate member 2220. The first elongate member 2218 and has a proximal end 2356, and a main body 2360 that defines a lumen 2362 through which the second elongate member 2320 and the wire member 2322 pass. In the illustrated embodiment, the proximal end 2356 of the first elongate member 2218 is attached to the orienting member 2214 within the first elongate member recess 2274. The first elongate member 2218 is moveable between a first, substantially straight configuration when the actuator 2216 is in the actuator first position and a second, curved configuration when the actuator 2216 is in the actuator second position.

In the illustrated embodiment, the second elongate member 2220 is rotatably attached to the housing 2212 and is rotatably attached to the first elongate member 2218. The second elongate member 2220 and has a lengthwise axis 2367, a proximal end 2368, and a tubular member 2372. The second elongate member 2220 is moveable between a first, substantially straight configuration when the actuator 2216 is in the actuator first position and a second, curved configuration when the actuator 2216 is in the actuator second position.

The tubular member 2372 of the second elongate member 2220 is rotatably attached to the housing 2212, extends through the housing 2212, extends through the passageway 2268 defined by the orienting member 2214, extends through the passageway 2328 defined by the main body 2316 of the actuator 2216, extends through the first passageway 2344 defined by the hub 2318, and extends through the lumen 2362 defined by the first elongate member 2218. The tubular member 2372 of the second elongate member 2220 has a proximal end 2380 and defines a lumen 2384 sized to receive a portion of a secondary medical device (e.g., lead extraction device, lead, locking stylet). In the illustrated embodiment, the proximal end 2380 of the tubular member 2372 is disposed inside the chamber 2238 defined by the housing 2212 and the housing 2212 defines a tubular projection 2239 that is coaxial with the tubular member 2372. In the illustrated embodiment, the tubular member 2372 has a first portion 2373 and a second portion 2375. The first portion 2373 is attached to the proximal end 2377 of the second portion 2375 and comprises a connector that accomplishes attachment between the second elongate member 2220 and the drive mechanism 2242.

In the illustrated embodiment, the wire member 2222 has a first end 2422 attached to the actuator 2216. The wire member 2222 is attached within the recess 2350 defined by the hub 2318 using the attachment member 2320. The wire member 2222 extends from the first end 2422, through a portion of the actuator 2216 (e.g., second passageway 2348 of the hub 2318 and passageway 2328 defined by main body 2316), between the first and second elongate members 2218, 2220 (e.g., within the lumen 2362 defined by the first elongate member 2218), and to a second end.

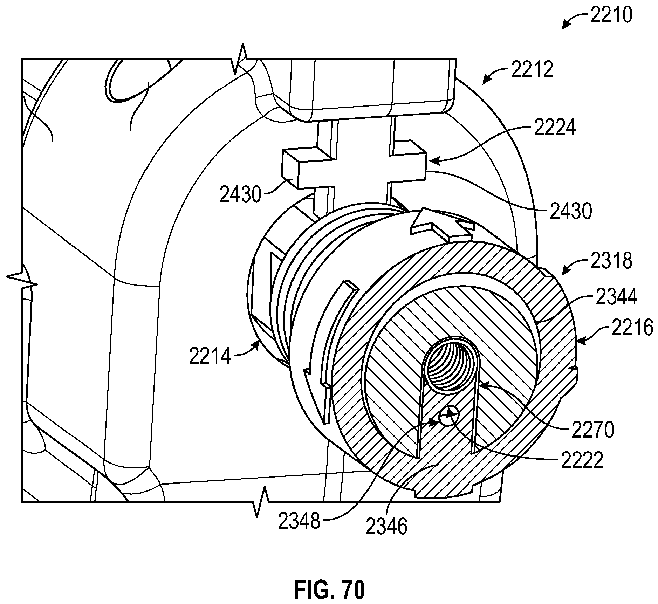

In the illustrated embodiment, the locking member 2224 is disposed through a passageway 2227 defined by the housing 2212 and is moveable between a first position, as shown in FIG. 68, in which the locking member 2224 is free of a recess of the plurality of locking member recesses 2272 defined by the orienting member 2214 and a second position, as shown in FIGS. 67 and 70, in which the locking member 2224 is disposed within a recess of the plurality of locking member recesses 2272 defined by the orienting member 2214. The orienting member 2214 and the hub 2318 can be rotated relative to the housing 2212 when the locking member 2224 is in the first position. The orienting member 2214 and the hub 2318 are fixed relative to the housing 2212 when the locking member 2224 is in the second position. As shown in FIGS. 67, 68, and 70, the locking member 2224 has a first end 2426, a second end 2428, and a plurality of projections 2430. The first end 2426 has a first width 2427 and the second end 2428 has a second width 2429 that is less than the first width 2427. Each projection of the plurality of projections 2430 is disposed between the passageway 2227 defined by the housing 2212 and the orienting member 2214 and prevents the locking member 2224 from becoming detached from the housing 2212.

While the deflectable catheter systems, medical devices, and their associated components, have been illustrated herein as having particular structural arrangements, other structural arrangements are considered suitable and a deflectable catheter system, medical device, and any associated component, can be formed of any suitable material and using any suitable technique or method of manufacture. Selection of a suitable structural arrangement, material, and/or suitable technique or method of manufacture can be based on various considerations, including the intended use of the deflectable catheter system and/or medical device. Examples of materials considered suitable to form a deflectable catheter system, medical device, and/or any component of a deflectable catheter system, include biocompatible materials, materials that can be made biocompatible, braided materials, coiled materials, metals, such as 316 stainless and 304 stainless, nitinol, corrosion resistant materials, plastics, polymers, polyethylene, such as high-density polyethylene (HDPE), polypropylene, polycarbonates, silicone, Delrin, transparent materials, opaque materials, combinations of the materials described herein, layered materials, and any other material considered suitable for a particular embodiment.

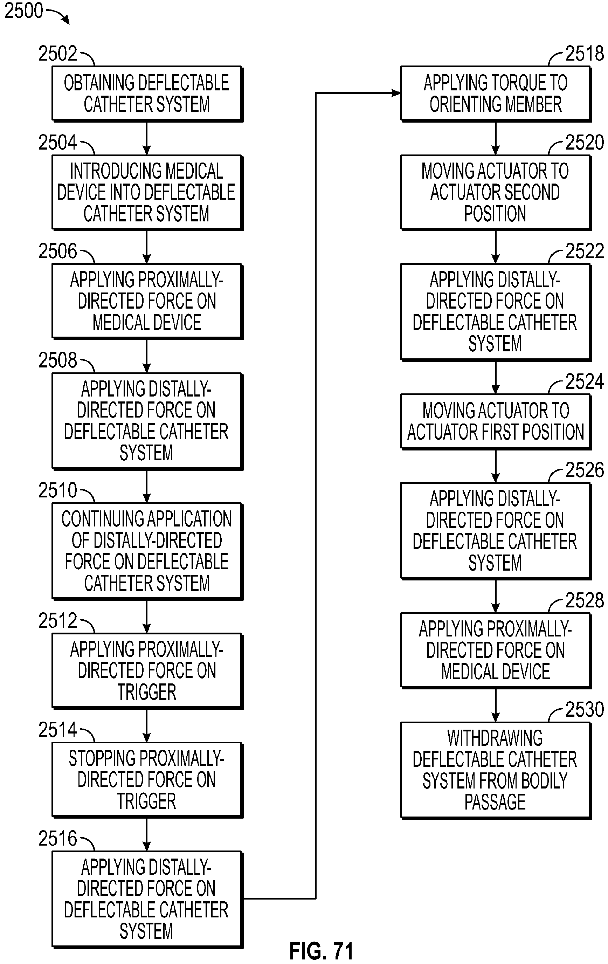

FIG. 71 illustrates another schematic illustration of an example method 2500 of using a deflectable catheter system. Method 2500 can be used to remove a medical device attached to a portion of a body of an animal, such as a human. The medical device can be disposed within a bodily passage of the body of the animal.

A step 2502 comprises obtaining a deflectable catheter system that has a housing, an orienting member, an actuator, a first elongate member, a second elongate member, and a wire member. Another step 2504 comprises introducing a portion of a medical device disposed within a bodily passage of a body of an animal into a lumen defined by the second elongate member. Another step 2506 comprises applying a proximally-directed force on the medical device while maintaining the position of the deflectable catheter system until the medical device is disposed proximal to the proximal end of the second elongate member. Another step 2508 comprises applying a distally-directed force on the deflectable catheter system while applying proximally-directed force on the medical device such that the deflectable catheter system is introduced into the bodily passage (e.g., vessel). Another step 2510 comprises continuing the application of a distally-directed force on the deflectable catheter system while applying proximally-directed force on the medical device such that the deflectable catheter system is advanced into the bodily passage. Another step 2512 comprises applying a proximally-directed force on the trigger while maintaining the position of the handle to rotate the second elongate member relative to the first elongate member and dissect tissue (e.g., encapsulated tissue) from the medical device. Another step 2514 comprises stopping the application of a proximally-directed force on the trigger. Another step 2516 comprises applying a distally-directed force on the deflectable catheter system while applying a proximally-directed force on the medical device such that the deflectable catheter system is advanced into the bodily passage. Another step 2518 comprises applying torque, or a force, on the orienting member while maintaining the position of the housing to move the orienting member, the first elongate member, and the wire member relative to the second elongate member. Another step 2520 comprises moving the actuator from the actuator first position to the actuator second position such that the first and second elongate members move from the first substantially, straight configuration to the second curved configuration. Another step 2522 comprises applying a distally-directed force on the deflectable catheter system while applying proximally-directed force on the medical device such that the deflectable catheter system is advanced into the bodily passage. Another step 2524 comprises moving the actuator from the actuator second position to the actuator first position such that the first and second elongate members move from the second, curved configuration to the first, substantially straight configuration. Another step 2526 comprises applying a distally-directed force on the deflectable catheter system while applying proximally-directed force on the medical device such that the deflectable catheter system is advanced to the distal end of the medical device. Another step 2528 comprises applying a proximally-directed force on the medical device while maintaining the position of the deflectable catheter system until the medical device is withdrawn from the bodily passage. Another step 2530 comprises applying a proximally-directed force on the deflectable catheter system until the deflectable catheter system (e.g., second elongate member) is withdrawn from the bodily passage.

Optional steps that can be completed prior to step 2502 when completing a superior approach (e.g., through the subclavian vein) include: surgically exposing a proximal end of the medical device; removing the medical device from any connections; removing any suture and/or tie-down materials attached to, or disposed over, the medical device; removing any proximal fittings attached to the medical device; introducing a wire guide into a lumen defined by the medical device; advancing the wire guide through lumen of the medical device to confirm patency of the lumen defined by the medical device; withdrawing the wire guide from the lumen defined by the medical device; introducing a locking stylet into the lumen defined by the medical device; advancing the locking stylet to the distal end of the medical device; locking the locking stylet in place; when the medical device comprises an active fixation type lead, another optional step comprises applying torque to the medical device to unscrew the medical device from tissue (e.g., cardiac tissue).

Step 2502 can be accomplished using any suitable deflectable catheter system, such as those described herein. For example, deflectable catheter system 610 illustrated in FIGS. 21 through 44 can be used. Alternatively, deflectable catheter system 10 illustrated in FIGS. 1 through 10, deflectable catheter system 310 illustrated in FIGS. 13 through 19, or a deflectable catheter system that includes one or more of the alternative components described herein can be used. In embodiments in which deflectable catheter system 10 is being used, step 504, step 506, step 508, and step 510, as described in method 500, can be completed prior to step 2502. In embodiments in which deflectable catheter system 310 is being used, step 510, as described in method 500, can be completed prior to step 2502.

Optionally, a deflectable catheter system can include a first stiffening member disposed within a second elongate member that defines a lumen through which method 2500 and method 500 can be accomplished (e.g., in place of the lumen defined by the second elongate member). The first stiffening member can have any suitable length, such as a length equal to, or less than, the length of the second elongate member. Once a desired point within the body has been reached, the first stiffening member can be removed from within the lumen defined by the second elongate member and other steps completed. The inclusion of a stiffening member provides a mechanism for increasing pushability and rigidity of the deflectable catheter system during use. Optionally, once a first stiffening member has been a removed, a second stiffening member, that has a length that is less than the length of the first stiffening member or greater than the length of the first stiffening member, can be introduced into the lumen defined by the second elongate member to provide pushability and rigidity to the deflectable catheter system. Any suitable number of stiffening members can be used to complete treatment such that these optional steps can be repeated any suitable number of times. A stiffening member can be formed of any suitable material, such as the materials described herein (e.g., plastic) and have any suitable dimensions, such as dimensions equal to, greater than, less than, or about 5.9 millimeters for an outside diameter, 5 millimeters for an outside diameter, 5 millimeters for an inside diameter, 4 millimeters for an inside diameter, 5 centimeters less than the total length of a second elongate member, 10 centimeters less than the total length of a second elongate member, 15 centimeters less than the total length of a second elongate member, and 20 centimeters less than the total length of a second elongate member.

Step 2504 can be accomplished by inserting any suitable portion of any suitable medical device into a lumen defined by the second elongate member. A medical device used to complete method 2500 has a proximal end, a distal end, and defines a lumen within which multiple components are disposed. For example, a medical device can comprise a cardiac lead that extends from the proximal end, which can be disposed outside of a bodily passage (e.g., subclavian vein), through the bodily passage (e.g., subclavian vein, superior vena cava, and right cardiac chamber(s)), to the distal end. The distal end of the medical device is attached to tissue (e.g., cardiac tissue within right atrium, cardiac tissue within right ventricle). While a cardiac lead has been described as an example of a medical device that can be used to complete method 2500, the deflectable catheter systems described herein can be used on any suitable tubular member and/or medical device disposed within a body.

Alternatively, step 2506 can comprise applying a proximally-directed force on the medical device while applying a distally-directed force on the deflectable catheter system until the medical device is disposed proximal to the proximal end of the second elongate member or comprise applying a distally-directed force on the deflectable catheter system while maintaining the position of the medical device until the medical device is disposed proximal to the proximal end of the second elongate member.

Step 2508 can be accomplished by introducing the deflectable catheter system into any suitable bodily passage and such that the deflectable catheter system is tracked over the medical device, which is disposed within the lumen defined by the second elongate member, and the second elongate member is introduced into the bodily passage. Examples of bodily passages within which it is considered suitable to introduce a deflectable catheter system, such as those described herein, include veins, such as the subclavian vein, arteries, and any other bodily passage considered suitable for a particular embodiment.

Step 2510 can be accomplished by advancing the deflectable catheter system over the medical device and into any suitable bodily passage such that the second elongate member is advanced into the bodily passage. Examples of bodily passages within which it is considered suitable to advance a deflectable catheter system, such as those described herein, include the veins, such as the subclavian vein, the superior vena cava, arteries, the right atrium, the right ventricle, and any other bodily passage considered suitable for a particular embodiment. In embodiments in which the deflectable catheter system includes a third elongate member (e.g., third elongate member 624), an optional step that can be accomplished prior to step 2510 comprises applying a proximally-directed force on the third tubular member while maintaining the position of the deflectable catheter system such that it is advanced into the bodily passage (e.g., 4 centimeters). Subsequently, step 2510 can be accomplished while maintaining the position of the third elongate member.

Step 2512 can be accomplished in instances in which the deflectable catheter system encounters tissue encapsulation (e.g., calcification, fibrous tissue) while being tracked over the medical device. Alternatively, in embodiments in which tissue encapsulation is not encountered, step 2512 and step 2514 can be omitted from method 2500. An optional step that can be completed concurrently with step 2512 comprises applying a proximally-directed force on the deflectable catheter system or a distally-directed force on the deflectable catheter system. Optionally, step 2512 can be repeated any suitable number of times, such as when any additional tissue encapsulation and/or calcification is encountered. Another optional step that can be completed prior to, or concurrently with, step 2512 comprises maintaining the position of the portion of the medical device (e.g., lead) that extends proximal to the lumen of the second elongate member. This optional step can be accomplished using any suitable method or technique, such as using a hand of a user, by wrapping the portion of the medical device around an anchor member (e.g., post), by passing the portion of the medical device between an anchor member (e.g., between first and second posts) and then grasping the portion of the medical device between a first hand of a user and a handle, and/or by passing the portion of the medical device between an anchor member (e.g., between first and second posts) and then grasping the portion of the medical device between a second hand of a user and a housing of the deflectable catheter system (e.g., near an actuator, distal end of housing) such that a first hand of the user can be used to grasp the handle and the trigger.

Step 2516 can be accomplished as described above with respect to step 2510.

Optionally, step 2518 can be omitted from method 2500 in embodiments in which it is not desired to move the direction in which the curve will be defined by a deflectable catheter system, such as in step 2520. In embodiments in which deflectable catheter system 10 or deflectable catheter system 310 is being utilized, step 2518 can comprise applying a torque to the handle of the deflectable catheter such that the distal end of the first elongate member rotates within the bodily passage.

Step 2520 can be accomplished in instances in which the bodily passage within which the deflectable catheter system is disposed is tortuous and it is desired to direct the distal end of the first and second elongate members in a particular direction. Optionally, in embodiments in which the bodily passage is not tortuous, step 2518 and step 2520 can be omitted from method 2500.

Step 2522 can be accomplished as described above with respect to step 2510. Steps 2518, 2520, and 2522 provide a mechanism for improving navigation of the deflectable catheter system during use.

Step 2524 can be accomplished in instances in which it is desired to position the distal end of the first and second elongate members in the first, substantially straight configuration. Optionally, step 2524 can be omitted from method 2500.

Step 2526 can be accomplished as described above with respect to step 2510.

Alternatively, in embodiments in which the deflectable catheter system includes a third elongate member (e.g., third elongate member 624), step 2528 can be accomplished by applying a proximally-directed force on the medical device while maintaining the position of the deflectable catheter system and applying a distally-directed force on a third elongate member such that it contacts the tissue within which the medical device is attached (e.g., cardiac tissue) and provides counterattraction against the tissue.

Any of the steps described in method 500 and method 2500 can be accomplished while visualizing the medical device, deflectable catheter system, bodily passage, and/or tissue using any suitable technique or method of visualization. For example, any of the steps described in method 500 and method 2500 can be accomplished under fluoroscopic monitoring.

In summary, example deflectable catheter systems are described. An example deflectable catheter system includes a housing, an orienting member, an actuator, a first elongate member, a second elongate member, and a wire member. The housing defines a passageway. The orienting member is attached to the housing. The actuator is attached to the orienting member and is moveable between an actuator first position and an actuator second position. The first elongate member is attached to the orienting member and defines a lumen. The first elongate member is moveable between a first, substantially straight configuration when the actuator is in the actuator first position and a second, curved configuration when the actuator is in the actuator second position. The second elongate member is disposed through the passageway of the housing and extends through the lumen defined by the first elongate member. The second elongate member is rotatable relative to the first elongate member.

Those with ordinary skill in the art will appreciate that various modifications and alternatives for the described and illustrated embodiments can be developed in light of the overall teachings of the disclosure, and that the various elements and features of one example described and illustrated herein can be combined with various elements and features of another example without departing from the scope of the invention. Accordingly, the particular arrangement of elements and steps disclosed herein have been selected by the inventor(s) simply to describe and illustrate examples of the invention and are not intended to limit the scope of the invention or its protection, which is set out by the appended claims.

## Claims

1. A deflectable catheter system (10) comprising:
a housing (12) defining a passageway (32);
an orienting member (15) attached to the housing and moveable relative to the housing between an orienting member first position and an orienting member second position;
an actuator (26) attached to the orienting member and moveable between an actuator first position and an actuator second position;
a first elongate member (14) attached to the orienting member and defining a lumen (106), the first elongate member moveable between a first, substantially straight configuration when the actuator is in the actuator first position and a second, curved configuration when the actuator is in the actuator second position;
a second elongate member (214) disposed within the passageway defined by the housing and extending through the lumen defined by the first elongate member, the second elongate member rotatable relative to the first elongate member; and
a wire member (18) having a first end attached to the actuator and a second end attached to the first elongate member.

2. The deflectable catheter system of claim 1, wherein the actuator has a main body and a hub (42) disposed within the main body, the hub mating with the main body of the actuator such that movement of the actuator between the actuator first position and the actuator second position results in movement of the hub between a first position and a second position; and
wherein the first end of the wire member is attached to the hub.

3. The deflectable catheter system of claim 1 or 2, wherein the second elongate member comprises a tubular member (772), a distal tip (774), a ring member (776), and a retaining member (778), the tubular member having a distal end, disposed within the lumen defined by the first elongate member, and rotatable relative to the first elongate member, the distal tip attached the distal end of the tubular member and having a first end and a second end, the ring member rotatably disposed on the distal tip between the second end of the distal tip and the retaining member, the retaining member attached to the distal tip between the first end of the distal tip and the ring member.

4. The deflectable catheter system of claim 3, wherein the distal tip defines a plurality of teeth (796) at the second end of the distal tip and/or wherein the distal tip is rotatable relative to the first elongate member, and/or wherein the second end of the wire member is attached to the ring member.

5. The deflectable catheter system of any of claims 1 to 4, wherein the wire member is disposed between the first elongate member and the second elongate member.

6. The deflectable catheter system of any of claims 1 to 5, wherein the first elongate member has an inner member (930) and an outer member (934), the inner member comprising a tubular member formed of a first material, the outer member comprising a tubular member formed of a second material that is different than the first material, optionally wherein the inner member has a lubricious coating adjacent to the second elongate member and/or optionally wherein the outer member includes a braided material (932) disposed within the second material.

7. The deflectable catheter system of any of claims 1 to 6, wherein the second elongate member comprises a plurality of flat wire members formed as coils.

8. The deflectable catheter system of any of claims 1 to 7, further comprising a third elongate member (624) slideably disposed on the first elongate member.

9. The deflectable catheter system of any of claims 1 to 8, wherein the second elongate member is moveable between a first, substantially straight configuration when the actuator is in the actuator first position and a second, curved configuration when the actuator is in the actuator second position.

10. The deflectable catheter system of any of claims 1 to 9, wherein movement of the orienting member between the orienting member first position and the orienting second position results in rotation of the first elongate member and wire member relative to the second elongate member.

11. The deflectable catheter system of any of claims 1 to 10, wherein the actuator is rotatably attached to the orienting member.

12. The deflectable catheter system of any of claims 1 to 11, wherein the housing has a drive mechanism (642) and a trigger (632) moveable between a first position and a second position, the drive mechanism moveable between an inactive state when the trigger is in the first position and an active state when the trigger is moved between the first and second positions; and
wherein the second elongate member is rotatable relative to the housing and the first elongate member when the drive mechanism is in the active state.

13. The deflectable catheter system of any of claims 1 to 12, wherein the housing has an exterior surface and an anchor member (636), the anchor member having a first post (644) extending from the exterior surface of the housing and away from the actuator.

14. The deflectable catheter system of any of claims 1 to 13, wherein the housing has a lengthwise axis (651);
wherein the housing has a first portion (656) and a second portion (658), the first portion moveable relative to the second portion axially along the lengthwise axis of the housing between a housing first position and a housing second position;
wherein the distal end of the second elongate member is disposed outside of the lumen defined by the first elongate member when the first portion of the housing is in the housing first position; and
wherein the distal end of the second elongate member is entirely disposed within the lumen defined by the first elongate member when the first portion of the housing is in the housing second position.

15. A deflectable catheter system comprising:
a housing defining a passageway;
an orienting member attached to the housing and moveable relative to the housing between an orienting member first position and an orienting member second position;
an actuator attached to the orienting member and moveable between an actuator first position and an actuator second position, the actuator having a main body and a hub disposed within the main body, the hub mating with the main body of the actuator such that movement of the actuator between the actuator first position and the actuator second position results in movement of the hub between a first position and a second position;
a first elongate member attached to the orienting member and defining a lumen, the first elongate member moveable between a first, substantially straight configuration when the actuator is in the actuator first position and a second, curved configuration when the actuator is in the actuator second position;
a second elongate member disposed through the passageway defined by the housing and extending through the lumen defined by the first elongate member, the second elongate member rotatable relative to the housing and the first elongate member, the second elongate member moveable between a first, substantially straight configuration when the actuator is in the actuator first position and a second, curved configuration when the actuator is in the actuator second position; and
a wire member disposed between the first elongate member and the second elongate member, the wire member having a first end attached to the hub of the actuator and a second end attached to the first elongate member;
wherein movement of the orienting member between the orienting member first position and the orienting second position results in rotation of the first elongate member and wire member relative to the second elongate member.

16. A method of using a deflectable catheter system to retrieve a medical device, wherein the method is not a method of treatment of the human or animal body by surgery or therapy, the method comprising:
obtaining a deflectable catheter system comprising:
a housing having a trigger, a handle, and defining a passageway;
an orienting member attached to the housing and moveable relative to the housing between an orienting member first position and an orienting member second position;
an actuator attached to the orienting member and moveable between an actuator first position and an actuator second position;
a first elongate member attached to the orienting member and defining a lumen, the first elongate member moveable between a first, substantially straight configuration when the actuator is in the actuator first position and a second, curved configuration when the actuator is in the actuator second position;
a second elongate member disposed through the passageway defined by the housing and extending through the lumen defined by the first elongate member, the second elongate member rotatable relative to the housing and the first elongate member, the second elongate member moveable between a first, substantially straight configuration when the actuator is in the actuator first position and a second, curved configuration when the actuator is in the actuator second position, the second elongate member having a proximal end, a distal end having a plurality of teeth, and defining a lumen; and
a wire member having a first end attached to the actuator and a second end attached to the first elongate member;
wherein movement of the orienting member between the orienting member first position and the orienting second position results in rotation of the first elongate member and wire member relative to the second elongate member;
introducing a portion of said medical device into the lumen defined by the second elongate member;
applying a proximally-directed force on said medical device while maintaining the position of the deflectable catheter system until said medical device is disposed proximal to the proximal end of the second elongate member;
applying a distally-directed force on the deflectable catheter system while applying proximally-directed force on said medical device;
continuing the application of a distally-directed force on the deflectable catheter system while applying proximally-directed force on said medical device;
applying a proximally-directed force on the trigger while maintaining the position of the handle to rotate the second elongate member relative to the first elongate member;
stopping the application of a proximally-directed force on the trigger;
applying a distally-directed force on the deflectable catheter system while applying a proximally-directed force on said medical device;
applying torque to the orienting member while maintaining the position of the housing to move the orienting member, the first elongate member, and the wire member relative to the second elongate member;
moving the actuator from the actuator first position to the actuator second position such that each of the first and second elongate members move from the first substantially, straight configuration to the second curved configuration;
applying a distally-directed force on the deflectable catheter system while applying proximally-directed force on said medical device;
moving the actuator from the actuator second position to the actuator first position such that each of the first and second elongate members move from the second, curved configuration to the first substantially, straight configuration;
applying a distally-directed force on the deflectable catheter system while applying proximally-directed force on said medical device such that the second elongate member is advanced to the distal end of said medical device;
applying a proximally-directed force on said medical device while maintaining the position of the deflectable catheter system; and
applying a proximally-directed force on the deflectable catheter system.

## Patentansprüche

1. System mit ablenkbarem Katheter (10), umfassend:
ein Gehäuse (12), das einen Durchgang (32) definiert;
ein Ausrichtungselement (15), das an dem Gehäuse angebracht und bezogen auf das Gehäuse zwischen einer ersten Position des Ausrichtungselements und einer zweiten Position des Ausrichtungselements beweglich ist;
ein Betätigungselement (26), das an dem Ausrichtungselement angebracht und zwischen einer ersten Position des Betätigungselements und einer zweiten Position des Betätigungselements beweglich ist;
ein erstes längliches Element (14), das an dem Ausrichtungselement angebracht ist und ein Lumen (106) definiert, wobei das erste längliche Element zwischen einer ersten, im Wesentlichen geraden Auslegung, wenn das Betätigungselement in der ersten Position des Betätigungselements ist, und einer zweiten, gekrümmten Auslegung, wenn das Betätigungselement in der zweiten Position des Betätigungselements ist, beweglich ist;
ein zweites längliches Element (214), das in dem von dem Gehäuse definierten Durchgang angeordnet ist und sich durch das von dem ersten länglichen Element definierte Lumen erstreckt, wobei das zweite längliche Element bezogen auf das erste längliche Element rotierbar ist; und
ein Drahtelement (18) mit einem ersten Ende, das an dem Betätigungselement angebracht ist, und einem zweiten Ende, das an dem ersten länglichen Element angebracht ist.

2. System mit ablenkbarem Katheter nach Anspruch 1, wobei das Betätigungselement einen Hauptkörper und einen Hub (42) hat, der in dem Hauptkörper angeordnet ist, wobei der Hub mit dem Hauptkörper des Betätigungselements im Eingriff ist, sodass die Bewegung des Betätigungselements zwischen der ersten Position des Betätigungselements und der zweiten Position des Betätigungselements in einer Bewegung des Hubs zwischen einer ersten Position und einer zweiten Position resultiert; und
wobei das erste Ende des Drahtelements an dem Hub angebracht ist.

3. System mit ablenkbarem Katheter nach Anspruch 1 oder 2, wobei das zweite längliche Element ein röhrenförmiges Element (772), eine distale Spitze (774), ein Ringelement (776) und ein Halteelement (778) umfasst, wobei das röhrenförmige Element ein distales Ende hat, das in dem von dem ersten länglichen Element definierten Lumen angeordnet und bezogen auf das erste längliche Element rotierbar ist, die distale Spitze an dem distalen Ende des röhrenförmigen Elements angebracht ist und ein erstes Ende und ein zweites Ende hat, das Ringelement rotierbar an der distalen Spitze zwischen dem zweiten Ende der distalen Spitze und dem Halteelement angeordnet ist, das Halteelement an der distalen Spitze zwischen dem ersten Ende der distalen Spitze und dem Ringelement angebracht ist.

4. System mit ablenkbarem Katheter nach Anspruch 3, wobei die distale Spitze eine Vielzahl von Zähnen (796) an dem zweiten Ende der distalen Spitze definiert und/oder wobei die distale Spitze bezogen auf das erste längliche Element rotierbar ist und/oder wobei das zweite Ende des Drahtelements an dem Ringelement angebracht ist.

5. System mit ablenkbarem Katheter nach einem der Ansprüche 1 bis 4, wobei das Drahtelement zwischen dem ersten länglichen Element und dem zweiten länglichen Element angeordnet ist.

6. System mit ablenkbarem Katheter nach einem der Ansprüche 1 bis 5, wobei das erste längliche Element ein Innenelement (930) und ein Außenelement (934) hat, wobei das Innenelement ein röhrenförmiges Element umfasst, das aus einem ersten Material gebildet ist, wobei das Außenelement ein röhrenförmiges Element umfasst, das aus einem zweiten Material gebildet ist, das sich von dem ersten Material unterscheidet, wobei optional das Innenelement eine gleitfähige Beschichtung angrenzend an das zweite längliche Element hat und/oder wobei optional das Außenelement ein geflochtenes Material (932) aufweist, das in dem zweiten Material angeordnet ist.

7. System mit ablenkbarem Katheter nach einem der Ansprüche 1 bis 6, wobei das zweite längliche Element eine Vielzahl von Flachdrahtelementen umfasst, die als Spulen gebildet sind.

8. System mit ablenkbarem Katheter nach einem der Ansprüche 1 bis 7, ferner umfassend ein drittes längliches Element (624), das verschiebbar an dem ersten länglichen Element angeordnet ist.

9. System mit ablenkbarem Katheter nach einem der Ansprüche 1 bis 8, wobei das zweite längliche Element zwischen einer ersten, im Wesentlichen geraden Auslegung, wenn das Betätigungselement in der ersten Position des Betätigungselements ist, und einer zweiten, gekrümmten Auslegung, wenn das Betätigungselement in der zweiten Position des Betätigungselements ist, beweglich ist.

10. System mit ablenkbarem Katheter nach einem der Ansprüche 1 bis 9, wobei die Bewegung des Ausrichtungselements zwischen der ersten Position des Ausrichtungselements und der zweiten Position des Ausrichtungselements in der Rotation des ersten länglichen Elements und des Drahtelements bezogen auf das zweite längliche Element resultiert.

11. System mit ablenkbarem Katheter nach einem der Ansprüche 1 bis 10, wobei das Betätigungselement rotierbar an dem Ausrichtungselement angebracht ist.

12. System mit ablenkbarem Katheter nach einem der Ansprüche 1 bis 11, wobei das Gehäuse einen Antriebsmechanismus (642) und einen Auslöser (632) hat, der zwischen einer ersten Position und einer zweiten Position beweglich ist, wobei der Antriebsmechanismus zwischen einem inaktiven Zustand, wenn der Auslöser in der ersten Position ist, und einem aktiven Zustand, wenn der Auslöser zwischen der ersten und der zweiten Position bewegt wird, beweglich ist; und
wobei das zweite längliche Element bezogen auf das Gehäuse und das erste längliche Element rotierbar ist, wenn der Antriebsmechanismus in dem aktiven Zustand ist.

13. System mit ablenkbarem Katheter nach einem der Ansprüche 1 bis 12, wobei das Gehäuse eine Außenfläche und ein Ankerelement (636) hat, wobei das Ankerelement einen ersten Pfosten (644) hat, der sich von der Außenseite des Gehäuses und von dem Betätigungselement weg erstreckt.

14. System mit ablenkbarem Katheter nach einem der Ansprüche 1 bis 13, wobei das Gehäuse eine Längsachse (651) hat;
wobei das Gehäuse einen ersten Abschnitt (656) und einen zweiten Abschnitt (658) hat, der erste Abschnitt bezogen auf den zweiten Abschnitt axial entlang der Längsachse des Gehäuses zwischen einer ersten Position des Gehäuses und einer zweiten Position des Gehäuses beweglich ist;
wobei das distale Ende des zweiten länglichen Elements außerhalb des von dem ersten länglichen Element definierten Lumens angeordnet ist, wenn der erste Abschnitt des Gehäuses in der ersten Position des Gehäuses ist; und
wobei das distale Ende des zweiten länglichen Elements vollständig in dem von dem ersten länglichen Element definierten Lumen angeordnet ist, wenn der erste Abschnitt des Gehäuses in der zweiten Position des Gehäuses ist.

15. System mit ablenkbarem Katheter, umfassend:
ein Gehäuse, das einen Durchgang definiert;
ein Ausrichtungselement, das an dem Gehäuse angebracht und bezogen auf das Gehäuse zwischen einer ersten Position des Ausrichtungselements und einer zweiten Position des Ausrichtungselements beweglich ist;
ein Betätigungselement, das an dem Ausrichtungselement angebracht und zwischen einer ersten Position des Betätigungselements und einer zweiten Position des Betätigungselements beweglich ist, wobei das Betätigungselement einen Hauptkörper und einen Hub hat, der in dem Hauptkörper angeordnet ist, wobei der Hub mit dem Hauptkörper des Betätigungselements im Eingriff ist, sodass die Bewegung des Betätigungselements zwischen der ersten Position des Betätigungselements und der zweiten Position des Betätigungselements in einer Bewegung des Hubs zwischen einer ersten Position und einer zweiten Position resultiert;
ein erstes längliches Element, das an dem Ausrichtungselement angebracht ist und ein Lumen definiert, wobei das erste längliche Element zwischen einer ersten, im Wesentlichen geraden Auslegung, wenn das Betätigungselement in der ersten Position des Betätigungselements ist, und einer zweiten, gekrümmten Auslegung, wenn das Betätigungselement in der zweiten Position des Betätigungselements ist, beweglich ist;
ein zweites längliches Element, das durch den von dem Gehäuse definierten Durchgang angeordnet ist und sich durch das von dem ersten länglichen Element definierte Lumen erstreckt, das zweite längliche Element bezogen auf das Gehäuse und das erste längliche Element rotierbar ist, das zweite längliche Element zwischen einer ersten, im Wesentlichen geraden Auslegung beweglich ist, wenn das Betätigungselement in der ersten Position des Betätigungselements ist, und einer zweiten, gekrümmten Auslegung, wenn das Betätigungselement in der zweiten Position des Betätigungselements ist, beweglich ist; und
ein Drahtelement, das zwischen dem ersten länglichen Element und dem zweiten länglichen Element angeordnet ist, wobei das Drahtelement ein erstes Ende hat, das an dem Hub des Betätigungselements angebracht ist, und ein zweites Ende, das an dem ersten länglichen Element angebracht ist;
wobei die Bewegung des Ausrichtungselements zwischen der ersten Position des Ausrichtungselements und der zweiten Position des Ausrichtungselements in der Rotation des ersten länglichen Elements und des Drahtelements bezogen auf das zweite längliche Element resultiert.

16. Verfahren zur Verwendung eines Systems mit ablenkbarem Katheter, um eine medizinische Vorrichtung zurückzuholen, wobei das Verfahren kein Verfahren zur Behandlung des menschlichen oder tierischen Körpers durch eine Operation oder Therapie ist, wobei das Verfahren umfasst:
Erhalten eines Systems mit ablenkbarem Katheter, umfassend:
ein Gehäuse mit einem Auslöser, einem Griff, das einen Durchgang definiert;
ein Ausrichtungselement, das an dem Gehäuse angebracht und bezogen auf das Gehäuse zwischen einer ersten Position des Ausrichtungselements und einer zweiten Position des Ausrichtungselements beweglich ist;
ein Betätigungselement, das an dem Ausrichtungselement angebracht und zwischen einer ersten Position des Betätigungselements und einer zweiten Position des Betätigungselements beweglich ist;
ein erstes längliches Element, das an dem Ausrichtungselement angebracht ist und ein Lumen definiert, wobei das erste längliche Element zwischen einer ersten, im Wesentlichen geraden Auslegung, wenn das Betätigungselement in der ersten Position des Betätigungselements ist, und einer zweiten, gekrümmten Auslegung, wenn das Betätigungselement in der zweiten Position des Betätigungselements ist, beweglich ist;
ein zweites längliches Element, das durch den von dem Gehäuse definierten Durchgang angeordnet ist und sich durch das von dem ersten länglichen Element definierte Lumen erstreckt, das zweite längliche Element bezogen auf das Gehäuse und das erste längliche Element rotierbar ist, das zweite längliche Element zwischen einer ersten, im Wesentlichen geraden Auslegung, wenn das Betätigungselement in der ersten Position des Betätigungselements ist, und einer zweiten, gekrümmten Auslegung, wenn das Betätigungselement in der zweiten Position des Betätigungselements ist, beweglich ist, wobei das zweite längliche Element ein proximales Ende und ein distales Ende mit einer Vielzahl von Zähnen hat und ein Lumen definiert; und
ein Drahtelement mit einem ersten Ende, das an dem Betätigungselement angebracht ist, und einem zweiten Ende, das an dem ersten länglichen Element angebracht ist;
wobei die Bewegung des Ausrichtungselements zwischen der ersten Position des Ausrichtungselements und der zweiten Position des Ausrichtungselements in der Rotation des ersten länglichen Elements und des Drahtelements bezogen auf das zweite längliche Element resultiert;
Einführen eines Abschnitts der medizinischenVorrichtung in das von dem zweiten länglichen Element definierte Lumen;
Aufbringen einer proximal gerichteten Kraft auf die medizinische Vorrichtung und dabei Aufrechterhalten der Position des Systems mit ablenkbarem Katheter, bis die medizinische Vorrichtung proximal zu dem proximalen Ende des zweiten länglichen Elements angeordnet ist;
Aufbringen einer distal gerichteten Kraft auf das System mit ablenkbarem Katheter, während eine proximal gerichtete Kraft auf die medizinische Vorrichtung aufgebracht wird;
Fortsetzen des Aufbringens einer distal gerichteten Kraft auf das System mit ablenkbarem Katheter, während eine proximal gerichtete Kraft auf die medizinische Vorrichtung aufgebracht wird;
Aufbringen einer proximal gerichteten Kraft auf den Auslöser, während die Position des Griffs aufrechterhalten wird, um das zweite längliche Element bezogen auf das erste längliche Element zu rotieren;
Beenden der Aufbringung einer proximal gerichteten Kraft auf den Auslöser;
Aufbringen einer distal gerichteten Kraft auf das System mit ablenkbarem Katheter, während eine proximal gerichtete Kraft auf die medizinische Vorrichtung aufgebracht wird;
Aufbringen eines Drehmoments auf das Ausrichtungselement, während die Position des Gehäuses aufrechterhalten wird, um das Ausrichtungselement, das erste längliche Element und das Drahtelement bezogen auf das zweite längliche Element zu bewegen;
Bewegen des Betätigungselements aus der ersten Position des Betätigungselements in die zweite Position des Betätigungselements, sodass sich jedes des ersten und des zweiten länglichen Elements aus der ersten, im Wesentlichen geraden Auslegung in die zweite, gekrümmte Auslegung bewegt;
Aufbringen einer distal gerichteten Kraft auf das System mit ablenkbarem Katheter, während eine proximal gerichtete Kraft auf die medizinische Vorrichtung aufgebracht wird;
Bewegen des Betätigungselements aus der zweiten Position des Betätigungselements in die erste Position des Betätigungselements, sodass sich jedes des ersten und des zweiten länglichen Elements aus der zweiten, gekrümmten Auslegung in die erste, im Wesentlichen gerade Auslegung bewegt;
Aufbringen einer distal gerichteten Kraft auf das System mit ablenkbarem Katheter, während eine proximal gerichtete Kraft auf die medizinische Vorrichtung aufgebracht wird, sodass das zweite längliche Element zu dem distalen Ende der medizinischen Vorrichtung vorgeschoben wird;
Aufbringen einer proximal gerichteten Kraft auf die medizinische Vorrichtung, während die Position des Systems mit ablenkbarem Katheter aufrechterhalten wird; und
Aufbringen einer proximal gerichteten Kraft auf das System mit ablenkbarem Katheter.

## Revendications

1. Système de cathéter déviable (10) comprenant :
un boîtier (12) définissant un passage (32) ;
un élément d'orientation (15) fixé au boîtier et mobile par rapport au boîtier entre une première position d'élément d'orientation et une seconde position d'élément d'orientation ;
un actionneur (26) fixé à l'élément d'orientation et mobile entre une première position d'actionneur et une seconde position d'actionneur ;
un premier élément allongé (14) fixé à l'élément d'orientation et définissant une lumière (106), le premier élément allongé étant mobile entre une première configuration sensiblement droite lorsque l'actionneur est dans la première position d'actionneur et une seconde configuration incurvée lorsque l'actionneur est dans la seconde position d'actionneur ;
un deuxième élément allongé (214) disposé à l'intérieur du passage défini par le boîtier et s'étendant à travers la lumière définie par le premier élément allongé, le deuxième élément allongé pouvant tourner par rapport au premier élément allongé ; et
un élément filaire (18) présentant une première extrémité fixée à l'actionneur et une seconde extrémité fixée au premier élément allongé.

2. Système de cathéter déviable selon la revendication 1, dans lequel l'actionneur a un corps principal et une embase (42) disposée dans le corps principal, l'embase s'accouplant avec le corps principal de l'actionneur de telle sorte qu'un mouvement de l'actionneur entre la première position d'actionneur et la seconde position d'actionneur entraîne un mouvement de l'embase entre une première position et une seconde position ; et
dans lequel la première extrémité de l'élément filaire est fixée à l'embase.

3. Système de cathéter déviable selon la revendication 1 ou 2, dans lequel le deuxième élément allongé comprend un élément tubulaire (772), une pointe distale (774), un élément annulaire (776) et un élément de retenue (778), l'élément tubulaire présentant une extrémité distale, disposée à l'intérieur de la lumière définie par le premier élément allongé, et pouvant tourner par rapport au premier élément allongé, la pointe distale étant fixée à l'extrémité distale de l'élément tubulaire et présentant une première extrémité et une seconde extrémité, l'élément annulaire étant disposé en rotation sur la pointe distale entre la seconde extrémité de la pointe distale et l'élément de retenue, l'élément de retenue étant fixé à la pointe distale entre la première extrémité de la pointe distale et l'élément annulaire.

4. Système de cathéter déviable selon la revendication 3, dans lequel la pointe distale définit une pluralité de dents (796) au niveau de la seconde extrémité de la pointe distale et/ou dans lequel la pointe distale peut tourner par rapport au premier élément allongé, et/ou dans lequel la seconde extrémité de l'élément filaire est fixée à l'élément annulaire.

5. Système de cathéter déviable selon l'une quelconque des revendications 1 à 4, dans lequel l'élément filaire est disposé entre le premier élément allongé et le deuxième élément allongé.

6. Système de cathéter déviable selon l'une quelconque des revendications 1 à 5, dans lequel le premier élément allongé présente un élément interne (930) et un élément externe (934), l'élément interne comprenant un élément tubulaire formé d'un premier matériau, l'élément externe comprenant un élément tubulaire formé d'un second matériau qui est différent du premier matériau, facultativement dans lequel l'élément interne présente un revêtement lubrifiant adjacent au deuxième élément allongé et/ou facultativement dans lequel l'élément externe comporte un matériau tressé (932) disposé à l'intérieur du second matériau.

7. Système de cathéter déviable selon l'une quelconque des revendications 1 à 6, dans lequel le deuxième élément allongé comprend une pluralité d'éléments filaires plats en forme de bobines.

8. Système de cathéter déviable selon l'une quelconque des revendications 1 à 7, comprenant en outre un troisième élément allongé (624) disposé de manière coulissante sur le premier élément allongé.

9. Système de cathéter déviable selon l'une quelconque des revendications 1 à 8, dans lequel le deuxième élément allongé est mobile entre une première configuration sensiblement droite lorsque l'actionneur est dans la première position d'actionneur et une seconde configuration incurvée lorsque l'actionneur est dans la seconde position d'actionneur.

10. Système de cathéter déviable selon l'une quelconque des revendications 1 à 9, dans lequel un mouvement de l'élément d'orientation entre la première position d'élément d'orientation et la seconde position d'orientation entraîne une rotation du premier élément allongé et de l'élément filaire par rapport au deuxième élément allongé.

11. Système de cathéter déviable selon l'une quelconque des revendications 1 à 10, dans lequel l'actionneur est fixé en rotation à l'élément d'orientation.

12. Système de cathéter déviable selon l'une quelconque des revendications 1 à 11, dans lequel le boîtier présente un mécanisme d'entraînement (642) et une gâchette (632) mobile entre une première position et une seconde position, le mécanisme d'entraînement étant mobile entre un état inactif lorsque la gâchette est dans la première position et un état actif lorsque la gâchette est déplacée entre les première et seconde positions ; et
dans lequel le deuxième élément allongé peut tourner par rapport au boîtier et au premier élément allongé lorsque le mécanisme d'entraînement est à l'état actif.

13. Système de cathéter déviable selon l'une quelconque des revendications 1 à 12, dans lequel le boîtier présente une surface extérieure et un élément d'ancrage (636), l'élément d'ancrage présentant un premier montant (644) s'étendant à partir de la surface extérieure du boîtier et à l'écart de l'actionneur.

14. Système de cathéter déviable selon l'une quelconque des revendications 1 à 13, dans lequel le boîtier présente un axe longitudinal (651) ;
dans lequel le boîtier présente une première partie (656) et une seconde partie (658), la première partie étant mobile par rapport à la seconde partie axialement le long de l'axe longitudinal du boîtier entre une première position du boîtier et une seconde position du boîtier ;
dans lequel l'extrémité distale du deuxième élément allongé est disposée à l'extérieur de la lumière définie par le premier élément allongé lorsque la première partie du boîtier est dans la première position du boîtier ; et
dans lequel l'extrémité distale du deuxième élément allongé est entièrement disposée à l'intérieur de la lumière définie par le premier élément allongé lorsque la première partie du boîtier est dans la seconde position du boîtier.

15. Système de cathéter déviable comprenant :
un boîtier définissant un passage ;
un élément d'orientation fixé au boîtier et mobile par rapport au boîtier entre une première position d'élément d'orientation et une seconde position d'élément d'orientation ;
un actionneur fixé à l'élément d'orientation et mobile entre une première position d'actionneur et une seconde position d'actionneur, l'actionneur présentant un corps principal et une embase disposée dans le corps principal, l'embase s'accouplant avec le corps principal de l'actionneur de telle sorte qu'un mouvement de l'actionneur entre la première position d'actionneur et la seconde position d'actionneur entraîne un mouvement de l'embase entre une première position et une seconde position ;
un premier élément allongé fixé à l'élément d'orientation et définissant une lumière, le premier élément allongé étant mobile entre une première configuration sensiblement droite lorsque l'actionneur est dans la première position d'actionneur et une seconde configuration incurvée lorsque l'actionneur est dans la seconde position d'actionneur ;
un deuxième élément allongé disposé à travers le passage défini par le boîtier et s'étendant à travers la lumière définie par le premier élément allongé, le deuxième élément allongé pouvant tourner par rapport au boîtier et au premier élément allongé, le deuxième élément allongé étant mobile entre une première configuration sensiblement droite lorsque l'actionneur est dans la première position d'actionneur et une seconde configuration incurvée lorsque l'actionneur est dans la seconde position d'actionneur ; et
un élément filaire disposé entre le premier élément allongé et le deuxième élément allongé, l'élément filaire présentant une première extrémité fixée à l'embase de l'actionneur et une seconde extrémité fixée au premier élément allongé ;
dans lequel un mouvement de l'élément d'orientation entre la première position d'élément d'orientation et la seconde position d'élément d'orientation entraîne une rotation du premier élément allongé et de l'élément filaire par rapport au deuxième élément allongé.

16. Procédé d'utilisation d'un système de cathéter déviable pour récupérer un dispositif médical, le procédé n'étant pas un procédé de traitement du corps humain ou animal par chirurgie ou thérapie, le procédé comprenant :
l'obtention d'un système de cathéter déviable comprenant :
un boîtier présentant une gâchette, une poignée, et définissant un passage ;
un élément d'orientation fixé au boîtier et mobile par rapport au boîtier entre une première position d'élément d'orientation et une seconde position d'élément d'orientation ;
un actionneur fixé à l'élément d'orientation et mobile entre une première position d'actionneur et une seconde position d'actionneur ;
un premier élément allongé fixé à l'élément d'orientation et définissant une lumière, le premier élément allongé étant mobile entre une première configuration sensiblement droite lorsque l'actionneur est dans la première position d'actionneur et une seconde configuration incurvée lorsque l'actionneur est dans la seconde position d'actionneur ;
un deuxième élément allongé disposé à travers le passage défini par le boîtier et s'étendant à travers la lumière définie par le premier élément allongé, le deuxième élément allongé pouvant tourner par rapport au boîtier et au premier élément allongé, le deuxième élément allongé étant mobile entre une première configuration sensiblement droite lorsque l'actionneur est dans la première position d'actionneur et une seconde configuration incurvée lorsque l'actionneur est dans la seconde position d'actionneur, le deuxième élément allongé présentant une extrémité proximale, une extrémité distale présentant une pluralité de dents, et définissant une lumière ; et
un élément filaire présentant une première extrémité fixée à l'actionneur et une seconde extrémité fixée au premier élément allongé ;
dans lequel un mouvement de l'élément d'orientation entre la première position d'élément d'orientation et la seconde position d'élément d'orientation entraîne une rotation du premier élément allongé et de l'élément filaire par rapport au deuxième élément allongé ;
l'introduction d'une partie dudit dispositif médical dans la lumière définie par le deuxième élément allongé ;
l'application d'une force dirigée proximalement sur ledit dispositif médical tout en maintenant la position du système de cathéter déviable jusqu'à ce que ledit dispositif médical soit disposé à proximité de l'extrémité proximale du deuxième élément allongé ;
l'application d'une force dirigée distalement sur le système de cathéter déviable tout en appliquant une force dirigée proximalement sur ledit dispositif médical ;
la poursuite de l'application d'une force dirigée distalement sur le système de cathéter déviable tout en appliquant une force dirigée proximalement sur ledit dispositif médical ;
l'application d'une force dirigée proximalement sur la gâchette tout en maintenant la position de la poignée pour faire tourner le deuxième élément allongé par rapport au premier élément allongé ;
l'arrêt de l'application d'une force dirigée proximalement sur la gâchette ;
l'application d'une force orientée distalement sur le système de cathéter déviable tout en appliquant une force orientée proximalement sur ledit dispositif médical ;
l'application d'un couple à l'élément d'orientation tout en maintenant la position du boîtier pour déplacer l'élément d'orientation, le premier élément allongé et l'élément filaire par rapport au deuxième élément allongé ;
le déplacement de l'actionneur de la première position d'actionneur à la seconde position d'actionneur, de telle sorte que chacun des premier et deuxième éléments allongés passe de la première configuration sensiblement droite à la seconde configuration incurvée ;
l'application d'une force dirigée distalement sur le système de cathéter déviable tout en appliquant une force dirigée proximalement sur ledit dispositif médical ;
le déplacement de l'actionneur de la seconde position d'actionneur à la première position d'actionneur, de telle sorte que chacun des premier et deuxième éléments allongés passe de la seconde configuration incurvée à la première configuration sensiblement droite ;
l'application d'une force dirigée distalement sur le système de cathéter déviable tout en appliquant une force dirigée proximalement sur ledit dispositif médical de telle sorte que le deuxième élément allongé soit avancé jusqu'à l'extrémité distale dudit dispositif médical ;
l'application d'une force dirigée distalement sur ledit dispositif médical tout en maintenant la position du système de cathéter déviable ; et
l'application d'une force dirigée proximalement sur le système de cathéter déviable.
